# EUROPEAN PATENT SPECIFICATION

(11) **EP 1 748 297 B1**
(45) Date of publication and mention of the grant of the patent: **23.06.2010**
(21) Application number: 06386024.1
(22) Date of filing: 13.07.2006
(51) Int. Cl.: G01N 33/68

(54) **Method for analysing naturally occuring peptides and small proteins using mass spectrometry**
Massenspektrometrie-Analyse von natürlich vorkommender Peptide und kleiner Peptide
Procédé d'analyse des peptides d'origine naturelle et petits peptides par spectrométrie de masse

(30) Priority: 27.07.2005 GR 2005100392
(43) Date of publication of application: 31.01.2007
(73) Proprietor: Foundation for Biomedical Research of the Academy of Athens, 115 27 Athens (GR); Vougas, Constantinos, 15669 Papagou (GR); Tsangaris, George, 152 35 Vrilissia Attikis (GR)
(72) Inventor: Tsangaris, George, 152 35 Vrilissia Attikis (GR); Vougas, Constantinos, 156 69 Papagou (GR); Fountoulakis, Michael, 115 27 Athens (GR)
(74) Representative: Tefou-Fotea, Eleni

(56) References cited:
- CHERTOV OLEG ET AL: "Organic solvent extraction of proteins and peptides from serum as an effective sample preparation for detection and identification of biomarkers by mass spectrometry" PROTEOMICS, vol. 4, no. 4, April 2004 (2004-04), pages 1195-1203, XP008071195 ISSN: 1615-9853
- CHERTOV O ET AL: "ENRICHMENT OF LOW-MOLECULAR-WEIGHT PROTEINS FROM BIOFLUIDS FOR BIOMARKER DISCOVERY" EXPERT REVIEW OF PROTEOMICS, FUTURE DRUGS LTD., LONDON, GB, 2005, pages 139-145, XP008066919 ISSN: 1478-9450
- ZHOU MING ET AL: "An investigation into the human serum "interactome"." ELECTROPHORESIS. MAY 2004, vol. 25, no. 9, May 2004 (2004-05), pages 1289-1298, XP002408172 ISSN: 0173-0835

## Description

### TECHNICAL FIELD WHERE THE INVENTION IS ADDRESSED

This invention refers to a method for the separation and identification by means of mass spectrometry- of peptides and small proteins sized 1.000-10.000Da (1-10kDa) referred to as Naturally Occurring Peptides and Small Proteins (NOP/SP) present in biological fluids such as plasma, serum, amniotic fluid, cerebrospinal fluid, urine, saliva, etc.

By application of this method NOP/SP are separated from the proteins with Molecular Weight greater than 10kDa present in the biological fluids, without complicated and time consuming procedures such as fractionation and enrichment and are analyzed by mass spectrometry. This method provides the possibility for the analysis of the NOP/SP using MALDI-TOF mass spectrometry, which is fast and can be potentially automated. Furthermore the aforementioned analysis can be furtherly enhanced by means of NOP/SP quantitation. All these facts render this method suitable for the analysis of a great number of samples in a short amount of time and for application in the diagnostic field, either through qualitative or quantitative analysis of one or more NOP/SP in the context of specific biological markers indicative of disease or in the context of protein profile comparison between normal and diseased samples.

### C. UTILITY OF THE AFOREMENTIONED TECHNIQUE

### C.1. INTRODUCTION

### C.1.1. PROTEOMICS

The term «proteomics» was initially indicted in 1996 by Marc Wilkins and is originating from the abbreviation of the words protein and genome. This term describes the sum of the protein content of a biological system at a given time or state. Since that time methodologies and techniques such as bioinformatics and mass spectrometry allowing the analysis of proteins have evolved rapidly. Some of them such as 2D polyacrylamide gel electrophoresis (2D-PAGE) have come to be especially popular. Proteomics have been widely utilized in the academic field as well as within the pharmaceutical industry sector since there is a strong belief that it will be a leading research factor in the post-genomic era.

The main field of proteomics is the full analysis-mapping of the protein content of a sample. The established and general approach of the proteomic analysis includes four steps: protein isolation from the sample, protein separation, protein detection and finally protein identification. Apart from the aforementioned analytical techniques, the protein identification procedure includes specific software for image analysis of polyacrylamide gels which includes gel comparison, in-gel protein quantitation and data storage and management.

### C.1.2 PROTEOMIC METHODOLOGY

### C.1.2.A. Protein separation

The methodology applied for the protein isolation from a sample is sample dependant. It also depends on the procedure that will be used for protein separation and identification. In the case which the protein separation will occur with the well established and general approach of isoelectric focusing, the protein isolation end up in the proteins's solubilisation in a specific denaturation solution which mainly contains Urea up to 8Molar concentration and additionally could contain Theiourea, CHAPS, 1,4-DTT, Tris, EDTA and protease inhibitors. In the case of biological fluids the proteins are already in solution. The sample is centrifuged in order all the insoluble cellular material and other insoluble elements to be removed. Following that, sample containing 1 mg of total protein is diluted in an appropriate volume of the aforementioned denaturation solution prior to further treatment. Due to the fact that the protein concentration is dependent upon the biological fluid type, in the case of very dilute biological fluids such as amniotic fluid or urine the samples are concentrated after the centrifugation and prior to the dilution with the denaturation solution. Sample concentration can be achieved using protein precipitation, ultrafiltration etc.

In the case of solid state biological material (tissue, cells, biopsy samples etc.) the proteins need to be extracted into solution prior to any further steps. This is achieved through homogenization (mechanical pressure, ultrasound etc.) in a small volume either of the aforementioned denaturation solution or of other types of solutions. After that, the already mentioned steps of centrifugation and dilution in denaturation solution take place in order the sample to be prepared for further analysis.

Finally, for the isolation of specific protein groups from a biological sample, specific fraction techniques such as chromatography or immunoprecipitation can be applied prior to further treatment.

### C.1.2.B. Protein separation using two dimensional acrylamide gel electrophoresis (2D-PAGE).

The general and classic method for the protein separation and detection used nowadays in proteomics is 2D SDS PAGE Gel Electrophoresis. The method is based on the concurrent isolation of proteins on the basis of their electric charge and molecular weight. The method results in the separation and visualization of hundreds of proteins at once, which is something that no other method is currently able to achieve. Initially the technique includes the isoelectric focusing (IEF) of proteins, meaning protein separation based on their electric charge, and finally their molecular weight based separation.

IEF is literally an equilibration process, during which proteins are forced to move along a strip carrying acrylamide derivatives (immobilines) of differential pH (IPG strip) under the effect of a powerful electric field. The proteins move and focus towards the points of the strip where their charge is neutralized (isoelectric point). There are IPG strips that cover a wide range of pH units such as pH 3-10 (length 7-24cm) as strips that are suitable for the analysis of very narrow pH ranges such as pH 3.5-4.5). One of the advantages of the IPG strips is the relatively large amount of protein sample that can be analyzed. The exact conditions of the IEF are dependant on a number of factors such as the quantity of the sample to be analyzed, sample application on the IPG strip, electric potential and isoelectric focusing duration.

The protein separation based on their molecular weight as a second dimension is achieved through the use of a polyacrylamide gel. This gel can be of constant or graded acrylamide concentration depending on the expected molecular weight of the proteins under study. The most commonly used gels are of constant concentration of 12% acrylamide and the gradient acrylamide concentration 9-16% for the separation of proteins of molecular weight range 10-200kDa.

### C.1.2.C. Protein Detection

After the protein separation in the acrylamide gel, proteins are detected through gel staining using sensitive stains the choice of which depends on the identification procedure that follows. The stains most commonly used are coomasie blue and silver stain. These stains are chosen when the mass spectrometry is going to be used for protein identification.

Given the proteinic heterogeneity in the 2D gels, the proteins are usually represented by more than one spots resulting in a number of translated gene products far greater than the number of the actual genes encoding them. In eukaryotes has been observed that it is possible for a single gene to encode 5-20 different proteins.

### C.1.2.D. Mass Spectrometry

The high throughput identification of proteins is achieved by mass spectrometry. Protein spots are removed mechanically from the 2D gel, they are distained, treated with trypsin (in-gel) and the resulting peptides are extracted into solution. The extracted peptides are analyzed by mass spectrometry for their mass and amino acid sequence to be determined.

Mass spectrometry comprises several steps for producing the desired measurements such as peptide ionization, release of these ionized peptides into the gas phase and high accuracy measurement of their mass. The ionization process and the release of the ions in the gas phase can be achieved with : a) Matrix Assisted Laser Desorption lonisation (MALDI), b) Surface Enhanced Laser Desorption lonisation (SELDI) and c) Electrospray lonisation (ESI). Following that the mass measurement can be achieved by: a) Measurement of the time of flight of the ions in a high vacuum tube (TOF), b) using an ion trap (IT), c) using quadrapoles and d) using a Fourier Transform system. Combining the ionization and mass measurement technologies the following techniques, which are widely used nowadays, accrue: a) MALDI-TOF, b) SELDI-TOF, c) ESI-TOF, d) ESI-IT, e) ESI-FT, f) ESI-IT-FT, g) ESI-QqTOF, h) ESI-QqQ etc.

According to the aforementioned techniques the peptide mass determination occurs by measuring the time of flight of the peptides in high vacuum tubes, while further analysis includes the deduction of the amino acid sequence of these peptides. Using highly specialized software the mass of the detected peptides is compared with the theoretical mass of all the peptides resulting from all the proteins of all known organisms which are indexed in various proteomic and genomic database and the protein identification is derived using highly sophisticated probabilistic models. This identification is usually based on more than five peptides which can be more that 20 in several instances, especially when the protein under study is of high molecular weight, while the probability of an error being less than 10⁻⁶, depending on the number of peptides that where used for the protein identification. Even more reliable results can be acquired by deriving the amino acid sequence of the analyzed peptides through the techniques of MALDI-MS-MS and ESI-MS-MS. In that instance the protein identification occurs through the amino acid sequence of the peptides and the final chance of an error is less than 10⁻¹⁰ depending on the number pf peptides analyzed for the specific protein.

From the aforementioned techniques MALDI-TOF and SELDI-TOF are characterized as high throughput because of their ability to rapidly and reliably analyze hundreds of samples. Because of the fact that the MALDI-TOF technology does not require peptide separation, is especially sensitive to organic and inorganic contaminant ions. Although the MALDI-TOF technology has a very high resolving power combined with great sensitivity in the signal detection the presence of contaminants result in a high level of mass spectra background noise which reduce the significance of the collected signal. In order for the mass spectra being produced from the MALDI-TOF technique to be evaluated the peptide mixture crystallized on the steel target has to be free of contaminant to the extent that is possible and the quantity of the peptides that go into the gas phase to be at least two times more intense than the background noise (Signal/Noise=2).

The SELDI-TOF technique, concerning the separation of the molecules under analysis, is based on the affinity of those molecules to specific substrates (biochip). For this reason a great number of different stationary sorbents (hydrophobic, anionic cationic and metallic) are used aiming at the specific binding of proteins of interest on their surface. The main disadvantage of this method is that the instrument that is used for the mass measurement is of low resolving power and not sensitive enough to detect lots of important molecules that are present in very small amounts in the sample. Furthermore the specific (low abundance) molecules are likely not to adhere to the biochips which are used in the SELDI-TOF technique, but to be washed of the sorbents surface along with other molecules, thus losing valuable information. Finally the ESI techniques have the advantage of unambiguous protein identification through amino acid sequencing but are extremely time consuming since prior to mass measurement there is a sample fractionation step using liquid chromatography. This fractionation step causes the analysis duration for a single sample to be between 25 minutes to 3 hours, while using the MALDI-TOF or the SELDI-TOF technologies, 384 samples are being processed within 2 - 3 hours.

In proteomics all the aforementioned techniques can be used simultaneously and in a complementary way in order all the valuable information concerning proteome differentiation in a disease state in relation to a normal state to be retrieved. The identification of these differentiations can be used as biological markers and therapeutic targets.

### C.2. The Biological Fluids Proteome

The term biological fluids in general is used to describe fluids that can be obtained from an organism with invasive are non-invasive methods where all the cellular components have been removed from. Plasma, blood serum, amniotic fluid, cerebrospinal fluid, saliva, tears, gastric fluid and in general any liquid that can be obtained from the organism in normal or disease conditions is considered biological fluid. Biological fluid surround tissues or are derived from them and contain a huge number of different proteinic molecules (proteins, oligopeptides, polypeptides etc.). Plasma and serum from the peripheral blood are biological fluids especially rich in proteinic molecules and are estimated to contain about 10.000 different proteins being derived from the whole organism. Because of the variability of the proteinic content of these biological fluids it is statistically certain that within this content dwells information relevant to various physiological and pathological states of the organism. Therefore, the identification of various types of proteinic molecules could offer valuable information relevant to the organism's reactions to various stimuli and states, in depth comprehension of cellular and tissue malfunctions which lead to various diseases and last but not least new therapeutical targets.

Based on the above, the plasma proteome and the proteomes of the remaining biological fluids is currently considered to be one of the most challenging areas of research in proteomics mainly in respect to the detection and identification of disease specific biological markers and active molecules for diagnosis and cure of these diseases. The proteomic analysis of biological fluids is an especially difficult and complicated procedure, primarily because of the big number of different gene products present in the sample and additionally because of the high degree of variation in protein concentration which is more than thousands orders of magnitude. For an example of the above, albumin which is the most abundant protein in plasma has a concentration of 10⁻³g/ml, while other important protein such as hormones and cytokines are present at a concentration range of 10-⁹g/ml (ng/ml) to 10⁻¹²g/ml (pg/ml).

It is known that 97-99% of the total protein amount in plasma and other biological fluids is comprised by only 22 proteins which belong to seven main groups and are referred to as high abundant proteins. These are: albumin (35-45mg/ml), immunoglobulins G, A and M (12-18mg/ml), fibrinogen (2-6mg/ml), a-1 antitrypsin (2-5mg/ml), a-2 macroglobulin (2-4mg/ml), transferin (2-3mg/ml) and lipoproteins (2-8.5mg/ml). The remaining 1-3% of the amount of protein present in the biological fluids is comprised by the low abundant proteins which seem to be extremely important for a biological system and therefore present very high potential for research. The low abundant proteomic fraction is divided in three subgroups, the high molecular weight proteome which includes proteins with molecular weight greater that 70kDa, the medium molecular weight proteome which includes proteins with molecular weight in the range of 30 - 70 kDa and the low molecular weight proteome which includes proteins with molecular weight less than 30kDa.

The vast molecular variation of the low abundant proteins present in the biological fluids plus their low abundance make their detection and analysis a very tedious task. Several approaches have been adopted to tackle this problem such as fractionation, separation and concentration of the biological sample. The greatest problem is caused by the high abundant proteins since their presence is masking the low abundance proteins inhibiting their detection. The obvious solution would be the removal of these high abundant proteins. Within that experimental frame several techniques have been utilized primarily aiming at the removal of albumin and immunoglobulins such as Cibacron dye, A/G Columns, antibodies, molecular weight cutoff filters, affinity chromatography, size exclusion chromatography etc. All these techniques have made the detection of the low abundant proteome possible. Nevertheless a very important fact needs to be considered. Albumin and other high abundance proteins such as a-2 macroglobulin are carrier proteins. This means that they bind and carry a great number of low abundant proteins. The removal of these carrier proteins would also mean the removal of all the proteins which are being carried, most of which are low abundant and of extreme interest. For instance the antimicrobial peptide a-defensin circulates in plasma being bound to albumin, a-2 macroglobulin and several serpins and is removed during high abundant protein depletion procedures.

### C.3. The low molecular weight proteome of biological fluids.

The biological fluids low molecular weight proteome includes a huge variety of proteinic molecules and peptides with molecular weight less than 30kDa. The low molecular weight proteome includes the small proteins with molecular weight more than 10kDa and the naturally occurring peptides with molecular weight less than 10kDa. The naturally occurring peptide fraction includes peptides coming from protein degradation, processing of immature protein precursors, and from the post-transcriptional intra and extra cellular proteinic alterations. All these processes operate within the complicated biological systems, offering additional functionality to the proteinic molecules, since the intact protein as well as its fractions can be utilized in many different metabolic pathways and cellular processes. A typical example of the above statement is the gene of pro-opio-melanocortin and the precursor protein that is produced by it, which after the post-translational, intracellular processing produces ten different peptidic hormones. Naturally occurring peptides and small proteins drive through tissues and cellular formations easily because of their small size and diffuse in the peripheral blood. Additionally since these peptides are relatively resistant to proteolytic degradation the have a long half-life. These two characteristics render naturally occurring peptides easily accessible and detectable, therefore good candidates for biological markers. Relevant studies in the human plasma and serum have illustrated that small proteinic molecules are directly related to diseases such as cancer, diabetes and cardiovascular diseases as well as other infectious diseases. More specifically, using the SELDI-TOF MS in conjunction with bioinformatic tools for protein profile comparison, five proteinic molecules were detected that were directly related to ovarian and prostate cancers. Four of these proteinic molecules had a molecular weight less that 2.500Da and the last one had a molecular weight of 3.080Da. These finding imply that the biological fluids low molecular weight proteome contains a very important deposit of histological information and can provide useful biological markers for the diagnosis of various diseases.

### C.3.A. Methods for the separation, detection and identification of the naturally occurring peptides and small proteins in biological fluids.

Naturally occurring peptides and small proteins belong to the low molecular weight proteome of the low abundant protein fraction of biological fluids. Furthermore these molecules are characterized by small molecular weights (<10kDa) and cannot be separated detected and identified with the classic wide use proteomic techniques as described in Chapter C.1.2. since the molecular weight range of the proteins that can be analyzed with these techniques fluctuates between 10 and 200kDa. There are several techniques that have been used for the identification of proteins belonging to the low molecular weight proteome of the biological fluids, and mainly include various low abundant protein enrichment schemes in conjunction with mass spectrometric techniques. Even though these techniques are not solely used for the analysis of naturally occurring peptide and small proteins, they can successfully identify these molecules among other of higher size. The methods currently used can be separated in three major groups: **a)** the low molecular weight proteome is separated by centrifugal ultrafiltration using filters of specific molecular weight cut-off, **b)** the low molecular weight proteome is separated-enriched by sample treatment with 2 volumes of pure acetonitrile and **c)** the naturally occurring peptides and small proteins of the plasma or the blood serum are fractionated in 96 fraction by liquid chromatography and are subsequently analyzed with mass spectrometry. In all the case the identification of the proteinic molecules is achieved via the combined use of liquid chromatography and mass spectrometry. Because of the last fact none of these methods are high throughput. Furthermore, since the concentration of the naturally occurring peptides and small proteins is quite low, incorporating multiple processing steps can result in loss of valuable information.

**C.3.A.1. Fist methodological approach:** Characterization of the low molecular weight human serum proteome. [Tirumalai RS, Chan KC, Prieto DA, Issaq HJ, Conrads TP, Veenstra TD. Mol Cell Proteomics 2003, 2: 1096-1103].

In this paper a methodology was developed for depletion of the high and medium molecular weight proteins (>30kDa) from human serum without loss of the low molecular weight fraction. This approach begins with serum centrifugal ultrafiltration using filters with molecular weight cut off greater than 30kDa. At the same time a specific solvent was used (20% acetonitrile) in order for the protein-protein binding to be dissociated. That has as a result the release of the naturally occurring peptides and small proteins from the carrier proteins such as albumin into solution. Following that the low molecular weight proteome is treated with trypsin and the resulting mixture of peptides is furtherly fractionated with strong cation-exchange chromatography. Each one of these fractions is furtherly fractionated by microcapillary reversed-phase liquid chromatography which is coupled to an ESI mass spectrometer (µLC-MS/MS). By using bioinformatics to analyze the whole amount of data produced 341 proteins of the human serum were identified.

Although the number of identified proteins is indicative of the method efficiency, it seems that this specific methodology approach is not efficient for the analysis of the naturally occurring peptides and small proteins with molecular weight less that 10kDa, since only 3 proteins with molecular weight less that 3.5kDa were identified. Because of sample trypsinisation, it was not possible for human serum naturally occurring peptides to be analyzed in their natural state. Finally this method is extremely time consuming since for the analysis of only 1 fraction using µLC-MS/MS, 120 minutes were required. Therefore this method is characterized as low-throughput. Because of the successive chromatography steps it is impossible for two samples to be analyzed simultaneously and in parallel.

### Method:

Centriplus centrifuge filters with molecular weight cut off 30kDa were used. The filter membranes were washed and used according to the manufacturer's instructions. 10ml of human serum were diluted with 50ml aqueous solution 25mM NH₄HCO₃ (pH 8.2), 20% (v/v) acetonitrile and were applied to the filter. The sample was centrifuged at 3.000g until 90% of the total sample volume passed through the filter. The filtrate was lyophilized to dryness and was resuspended in 1ml 25mM NH₄HCO₃ pH 8.2. Following that the sample was denatured by boiling for 5 minutes. The sample was allowed to cool-down to room temperature. Following that it was reduced using 5mM dithiotheitol and heating up to 56°C fro 1 hour. The sample was then alcylated using 10mM iodoacetamide at 25°C for 1 hour. Trypsin was added to the sample at a ratio protein:enzyme=50:1 and it was allowed to act for 16-18 hours (overnight) at 37°C. After the incubation trypsin was inactivated by acidifying the sample using trifluoroacetic acid at a final concentration of 0.1%. The sample was desalted using the reversed phase solid phase extraction column Bond Elut C-18. The column eluent was again lyophilised to dryness and was resuspended in 500µl deionised and double distilled water. 5µl of sample were loaded in the µLC and the peptides were eluted into the mass spectrometer under an organic solvent gradient (5%-85%) spanning 100 minutes at a flow rate of 0.5µl/min.

**C.3.A.2. Second Methodological approach:** Analysis of Low molecular weight human serum proteome using ultrafiltration, isoelectric focusing and mass spectrometry. [Harper RG, Workman SR, Schuetzner S, Timperman AT, Sutton JN. Electrophoresis 2004, 25: 1299-1306].

This paper presented a modification and optimization of the first method (Chapter C.3.A.1.) for the analysis of the human serum low molecular weight proteome. The protein identification was accomplished by the use of centrifugal ultrafiltration, isoelectric focusing, off-line multidimensional chromatographic separations and mass spectrometry (MS/MS). The result of this approach was the identification of 262 proteins that were considered to belong to the human serum low molecular weight proteome. Like the previous method, this one has the disadvantages regarding the identification of the naturally occurring peptides and small proteins, since the use of the enzymatic digest rules out the analysis of such molecules as naturally occurring for the reasons that have been already mentioned. Furthermore the analysis time of this approach is long (235 minutes per enzyme, for 3 enzymes sums up to a total of 705 minutes for analysis of just one sample using mass spectrometry). These times make the method low throughput.

### Method:

Micron centrifugal filters with a molecular weight cutoff of 50kDa were used. The filters were washed and used according to the manufacturer's guidelines. 1ml of human serum was aliquoted in 200µl aliquots. Each aliquot was diluted in 120µl of 20% Acetonitrile in deionised water and was heated up to 45°C for 15minutes in order for the proteins to be denatured. The diluted samples were centrifuged at 14.000g for 10minutes for the insoluble material that would block the filters to be removed. The supernatants were loaded in the aforementioned filters and were centrifuged at 2.000g for 23 and 90 minutes (total time of 113minutes).

The low molecular weight proteins were fractionated in a miniscale isoelectric focusing unit as follows. All the solutions containing the low molecular weight proteins from the previous step were pooled and diluted in 18ml of a solution containing 1%Bio-Lyte 3-10 ampholytes, 8M Urea and 2mM DTT. The resulting solution containing the proteins was focused at 10W for 5hours at 4°C. 20 fractions were collected from this procedure. Same pH fractions were pooled and the proteins were acetone precipitated using 3 volumes of acetone to 1 volume of sample. The proteins were pelleted by centrifugation. Following that they were dried and resuspended in a solution containing 8M Urea, 100mM Tris-HCl pH 8.5. 30% of each pH fraction was loaded and run in SDS-PAGE Gels and the protein profile was visualized with the use of silver nitrate. The remaining of the fractions were enzymatically incubated. Prior to the enzymatic incubation the protein disulphide bonds were reduced through treatment with 5µl of a solution containing 100mM DTT at 57°C for 1 hour. Following that the samples were allowed to cool down and the free thioles were alcylated through treatment with 5µl of a solution containing 300mM lodoacetamide. The treated samples were separated in three equal volume aliquots and were incubated with three different proteases (trypsin, chymotrypsin and sabtilisin). The resulting peptides were analyzed using reversed phase chromatography which was coupled (on-line) to an ESI mass spectrometer (µLC-MS/MS). The total analysis time for each different enzymatic digest was 235 minutes.

**C.3.A.3. Third methodological approach:** Top-Down analysis of the low molecular weight human plasma proteome using hybrid lon-Trap-Fourier Transform Mass Spectrometry. [Sutton Jn, Bonilla LE, Richmond T, Gerszten RE, Liu E, Shi X, Senko M, Zabrouskov V, Kelleher N, Forbes A, Harper RG. Thermo Electron Corporation, Application Note: 344, 2005].

In the previous two methods the low molecular weight proteome was treated was enzymes and the proteins were identified through comparison of the resulting peptides spectra to in silico generated peptide spectra from protein sequences stored in databases. Top-Down method, on the contrary, allows the study of intact proteins without the need for an enzymatic digest. In this paper a new top-down approach was applied using the Thermo Electron Co mass spectrometer LTQ for the analysis of the human plasma low molecular weight proteome and peptidome. The aim of this method was to provide a protocol for enrichment and analysis using the LTQ FT mass spectrometer for the identification of intact human plasma low molecular weight proteins without the need for any enzymatic digest.

This method lacks the aforementioned disadvantage of the proteinic enzymatic digest prior to their identification. Therefore it has the ability to identify naturally occurring molecules present in human plasma. Nevertheless, from the methods applied, it is evident that the results are mainly due to the characteristics of the LTQ FT mass spectrometer and not due to the methods used since they closely resemble the already mentioned methods. Furthermore, the analysis time for one sample seems to be quite long while the application of chromatographic techniques eliminates the possibility for simultaneous treatment of many samples, rendering the methods as low throughput.

### Method:

The isolation of the low molecular weight proteome is performed with centrifugal ultrafiltration in a way very similar to the ones already mentioned with minor alterations. The filters used had molecular weight cut-offs of 30 and 50kDa and were treated according to the manufacturer's guidelines. 1ml of human serum was aliquoted in 200µl aliquots. Each aliquot was diluted in 120µl of 20% Acetonitrile in deionised water and was heated up to 40°C for 15minutes in order for the proteins to be denatured. The diluted samples were centrifuged at 10.000g for 10minutes for the isoluble material that would block the filters to be removed. The supernantants were loaded in the aforementioned filters and were centrifuged at 1.500g at 4°C for as long as it would require for almost one quarter of the applied volume to pass through the filter (-90minutes). All the filtrates were pooled and concentrated via lyophilisation. Aliquots from the concentrated samples were diluted in 100µl of 5% acetonitrile, 0.1% formic acid and the low molecular weight proteins were collected and concentrated with the use of a special HPLC Column (NPS-ODS I). The analysis of the low molecular weight proteome and peptidome was finally carried out with an Reversed Phase HPLC system couples on-line to the LTQ-FT. The peptide elution from the HPLC Column took place over a linear gradient profile which lasted 40 minutes.

**C.3.A.4. Fourth methodological approach:** Analysis of low-abundance, low-molecular weight serum proteins using mass spectrometry. [Merrell K, Southwick K, Graves SW, Esplin MS, Lewis NE, Thulin GD. Journal of Biomolecular Techniques 2004, 15:238-248].

In previous studies has been determined that treatment of a human plasma or serum volume with 4 to 5 volumes of pure acetonitrile is sufficient for the precipitation of the protein content of these biological fluids without further treatment (USA Patent number US4171204/1979). Furthermore, it has been determined that treatment of one volume of plasma or serum with 2 volumes of pure acetonitrile is the best method for the removal of albumin and immunoglobulins from plasma or serum because of the fact that the acetonitrile not only denatures these big proteins but also forces the small molecules that are bound on these big high abundant carrier proteins (such as albumin) to be released into solution being available for further study. In this paper one volume of serum was treated with two volumes pf HPLC grade acetonitrile. After the high abundance proteins precipitation using centrifugation, the small proteins and peptides that remained in the supernatant were analyzed by LC-MS. Using this approach around 4.000 different ion species below 2.5kDa as well as some small proteins between 2.5 and 10kDa were detected per serum sample. Furthermore in this paper the use of centrifugal filters and the use of pure acetonitrile as means for the analysis of the naturally occurring peptides and small proteins were compared. The writers claim that the use of acetonitrile in a volumetric ratio of 2 volumes of pure acetonitrile to 1 colume of sample is more effective than the use of ultrafiltration or dialysis for the analysis of low molecular weight proteinic molecules using LC-MS.

Nevertheless the time for the analysis seems to be quite long just as in all the previously mentioned methods, while the use of chromatographic separations is prohibitive for the parallel treatment of more than one samples at a time, rendering the technique as low throughput.

### Methods:

a) 2 volumes (400µl) acetonitrile HPLC grade were added to a volume of human serum (200µl). The mixture was shortly vortexed. The sample was lefton the bench to stand for 30 minutes and then it was centrifuged at 12.000rpm for 4 minutes. A fraction of the supernatant was concentrated through lyophilisation up to a volume of 50µl. The sample was brought back to the original volume of 200µl by addition of water.
b) 2 volumes (400µl) acetonitrile HPLC grade were added to a volume of human serum (200µl). The mixture was shortly vortexed. The sample was left on the bench to stand for 30 minutes and then it was centrifuged at 12.000rpm for 10 minutes at room temperature. 550µl from the supernatant was concentrated to 200µl using centrifugation under vacuum. The total protein amount present in the solution was deduced and solution volume containing 4µg of protein was transferred in a new microcentrifuge tube and was lyophilized almost to dryness. In the lyophilized sample 20µl of 88%formic acid, 2µl of mellitin (5pmol/µl) and 2µl of fibrinopeptide were added and the solution was brought to a final volume of 40µl with water.
c) 1ml of serum was diluted with 5ml of 25mM Ammonium Bicarbonate solution and was centrifugally filtrated using a filter of 30kDa molecular weight cut off. After the centrifugation the filtrate was lyophilized to dryness and was resuspended initially in 40µl (20µl 88% formic acid 20µl water) and finally was brought to the initial volume of 1ml with further addition of water. In 200µl of this solution 400µl of pure acetonitrile was added in order the proteins in solution to be precipitated.

For further analysis, 1µg of protein from the samples obtained by the afforemenioed procedures was analysed by LC-MS. The total analysis time for each sample was 55 minutes.

**C.3.A.5. Fifth methodological approach:** Organic solvent extraction of proteins and peptides from serum as an effective sample preparation for detection and identification of biomarkers by mass spectrometry. [Chertov O, Biragyn A, Kwak LW, Simpson JT, Boronina T, Hoang VM, Prieto DA, Conrads TP, Veensrta TD, Fisher RJ. Proteomics 2004,4: 1195-1203].

In this paper there was a method developed for the isolation of peptides and low molecular weight proteins from serum with the use of pure acetonitrile and 0.1% TFA. The approach used was practically the same with the one in the fourth approach (Chapter C.3.A.4.). The only difference was the addition of 0.1%TFA in pure acetonitrile. One volume of serum is mixed with 2 volumes of pure acetonitrile, 0.1%TFA and the mixture is centrifuged. The writers adjudge that the use of 2 volumes of pure acetonitrile containing 0.1% TFA satisfies both criteria for the analysis of the low molecular weight proteome: the high molecular weight high abundance proteins such as albumin are effectively remove via precipitation while peptides and small proteins remain in solution and are therefore available for further analysis.

Judging by the paper results, the analysis of the low molecular weight proteome was carried out using the SELDI-TOF technology, which did not allow (because of its limitations) the identification of the analyzed molecules. Some identification occur through the MALDI-TOF technology at a latter stage were the sample was fractionated with chromatography and proteins were enzymatically digested. This method has the disadvantage of low productivity since the identification of the molecules under study requires chromatographic separation.

### Methods:

a) 150µl of serum was mixed with 2 volumes (300µl) of poure acetonitrile, 0.1% TFA, resulting in a concrete pellet. The supernantant was transferred into a new tube and it was centrifuged at 10.000rpm for 5 minutes at 4°C. The new supernantant was lyophilised to dryness and was resuspended in 5% acetonitrile 0.1% TFA in water. HPLC fraction of the resuspended sample followed. Some of the obtained fractions were treated with trypsin and MALDI TOF analysis for peptide identification was performed.
b) In a volume of serum 2 volumes of pure acetonitrile containing 0.1% TFA were added and were mixed rapidly with vortexing. The sample was centrifuged at 10.000rpm for 5 minutes at 4°C. The supernantant was lyophilised to dryness. The supernantant was properly resuspended and SELDI-TOF or MALDI-TOF analysis was performed.

### D. Presentation of the invention as it is defined in the claims.

The present invention refers to a biological fluids treatment method which aims at the isolation and identification of the naturally occurring peptides with molecular weight ≤ 10kDa and small proteins (NOP/SP) of molecular weight less than 10kDa using mass spectrometry. NOP/SP as already mentioned belong to the low abundance low molecular weight proteome of the biological fluids. Apart from the low abundance of these molecules an additional problem for their detection is the fact that in biological fluids the small proteinic molecules are often attached to high molecular weight high abundance carrier proteins such as albumin. The NOP/SP isolation method has to facilitate their release in solution from these carrier proteins.

For the method development of the current invention the fact that the protein solubility is a result of polar interactions with the soluble face, ionic interactions with salts and electrostatic forces between charged molecules bearing the same charge, was taken into consideration. The disturbance of these interactions with the use of organic solvents, acids, salts or metallic ions causes the decrease of protein solubility and finally their precipitation from the soluble face of the biological fluids. The organic solvents, when used as protein precipitants, have double action. They decrease the protein solution dielectric constant, fact that favors the electrostatic interactions between proteinic molecules. Furthermore, they displace water molecules from the proteinic surface which causes the electrostatical interactions of hydrophobic interactions to prevail, causing protein aggregation and precipitation. Additionally proteins present their minimum solubility at their isoelectric point because of the fact that there is no net electric charge on the protein molecule. It has also been observed that acidic reagents cause the formation of insoluble salts with the positively charged protein amino groups in environments where the pH is less than their isoelectic point and thus low pH favors protein precipitation. Relevant studies have shown that the addition of pure acetonitrile in plasma in a volumetric ratio of 2:1 (2 volumes of acetonitrile to 1 volume of sample) precipitates a minimum of 92% of the total protein amount present while the use of acidic reagents such as TFA and formic acid not only has been shown to facilitate the protein precipitation but also seems to have an effect in the proteinic ionisation during their further analysis with LC-MS. [Optimisation of protein precipitation based upon effectiveness of protein removal and ionisation effect in liquid chromatography-tandem mass spectrometry. Polson C, Sarkar P, Incledon B, Raguvaran V, Grant R. Journal of Chromatography B 2003, 785: 263-275].

Taking all the above into consideration, in the current invention acetonitrile was used for the separation of NOP/SP from the rest of the proteins present in the biological fluids with molecular weight greater than 10kDa, primarily because of its effectiveness as protein precipitant and additionally because of its ability to disrupt protein-protein bonds, for NOP/SP to be released in solution. In contradiction to the up-to-date methodologies for the analysis of the low molecular weight proteome of the biological fluids, this invention's originality lies to the fact that with the use of aqueous solution containing 50% acetonitrile and 0.1% TFA, NOP/SP were separated for the remaining protein molecules of the biological fluids with molecular weight greater that 10kDa and their analysis as naturally occurring molecules using mass spectrometry became possible.

The choice of the specific acetonitrile-TFA solution was made for three reasons:
**a)** for the properties of acetonitrile regarding its ability to release the small proteinic molecules from carrier proteins to be utilized.
**b)** for the change of the solution dielectric constant and the protein molecules electrostatic interactions to be achieved.
**c)** to increase the available water molecules.

The result of the aforementioned choices is the isolation of NOP/SP from the remaining protein molecules of the biological fluids with molecular weight greater that 10kDa. The addition of the acetonitrile solution in biological fluids seems to disrupt the protein-protein bonds releasing NOP/SPs into solution. NOP/SPs because of the increase of the water molecules are stabilized in solution, while at the same time because of the solution dielectric constant change and the electrostatic interactions between the proteins of higher molecular weight, these proteins aggregate and precipitate. NOP/SPs because of their small size remain in solution.

### D.1. Method Presentation

### D.1.A. For the analysis of NOP/SP in biological fluids with high total protein concentration.

In a given volume (a) of a biological fluid sample, 7 to 9 volumes (7xa to 9xa) of 50% acetonitrile in water containing also 0.1% TFA, are added [acetonitrile and water are HPLC grade]. The mixture is vortexed for 5 seconds and is immediately centrifuged at 13.000rpm (15.000xg) for 30 minutes at 4°C. The supernatant is placed in a new tube and is concentrated with centrifugation under vacuum down to volume (b), which is appropriate and functional for the subsequent steps of the method.

Prior to analysis with mass spectrometry, the concentrated sample is desalted with the use of a pipette tip packed with C18 material. Prior to the desalting of the concentrated sample, the C18 material packed in the tip is washed with pure acetonitrile HPLC grade, then with 50%acetonitrile in water and is finally equilibrated with aqueous solution containing 3%acetonitrile and 0.1%TFA. After the equilibration step the concentrated sample passes through the material several times by pipetting up and down with an appropriate pipette. At that step the NOP/SP are bound on the C18 material. The sample is desalted by further washes with 3%acetonirile, 0.1%TFA. Finally NOP/SP are eluted from the C18 material using aqueous solution 60% acetonitrile, 0.3%TFA directly on a steel MALDI target. Matrix solution [0.3% (w/v)a-Cyano-4-hydroxycinamic acid, 50% (v/v)acetonitrile, 0.1%(v/v)TFA] is then added to the eluant and the mixture is allowed to dry. After the solution has dried and a spot has formed, matrix solution is again added and is allowed to dry. Following that procedure the MALDI target is inserted in the MALDI instrument for the analysis. Alternatively the eluant from the C18 material is placed on a specific MALDI target spot were matrix solution has previously been allowed to dry. After drying of the eluant matrix solution is again added and after it dried the target is placed in the instrument for the analysis.

For the analysis of the concentrated sample using an ESI-TOF mass spectrometer, the sample is desalted using pipette tip packed with C18 material using the previously described procedure the only difference being that instead of 0.1%TFA, 5% formic acid is used in all the solutions. Consequently the NOP/SP are eluted using aqueous solution 60% acetonitrile 5% formic acid and are collected in an eppendorf. A fraction from the eluant is then placed in a special glass tube with external metallic coating and small cross-section and is injected-infused into the mass spectrometer with pressure and application of high voltage.

### D.1.B. For the analysis of NOP/SP in biological fluids with low total protein concentration.

In the case a biological fluids with low total protein concentration the same procedure as with the biological fluids of high protein concentration is followed. The only difference is that the concentration step precedes the centrifugation step.

In a given (a) volume of biological fluid, 7 to 9 volumes of 50% acetonitrile in water containing also 0.1% TFA, are added [acetonitrile and water are HPLC grade]. The sample is immediately vortexed for 5 sec. The sample is concentrated using centrifugation under vacuum until the transparent solution becomes blur at a volume (b) which is appropriate and functional for the following steps. The concentrated solution is centrifuged at 13.000rpm (15.000xg) for 30 minutes at 4°C. After the centrifugation the supernatant is transferred to a new tube and the same procedure of desalting and analysis with mass spectrometry (MALDI-TOF, ESI_TOF etc.) as described above follows (C.1.A).

### D.2. Method Effectiveness

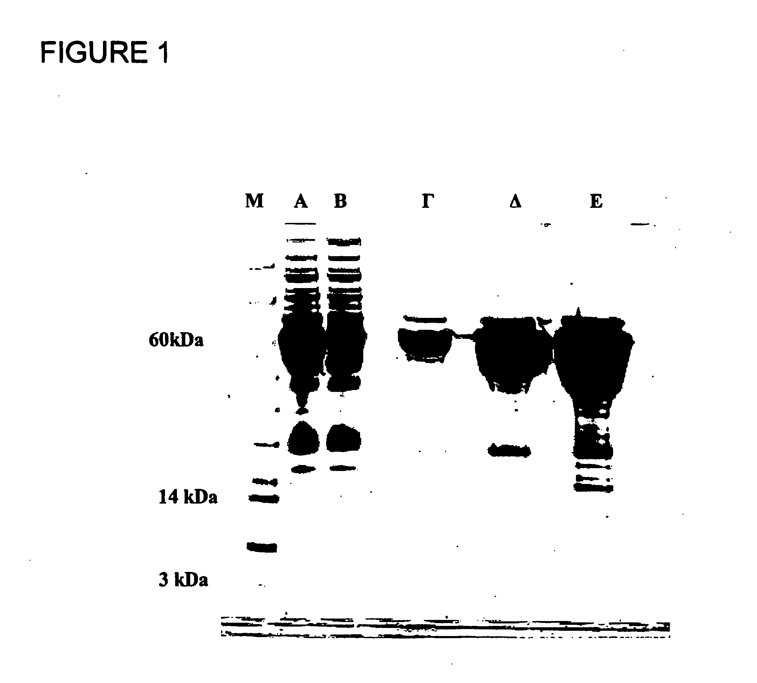

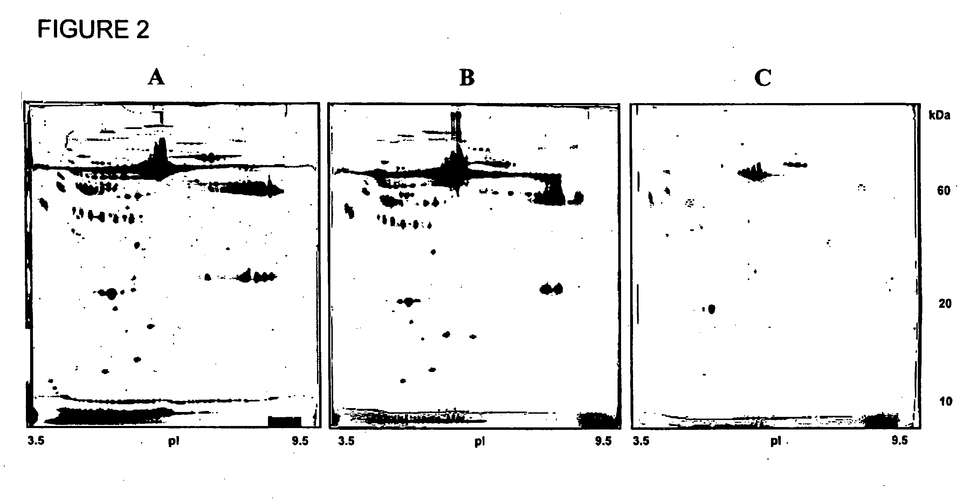

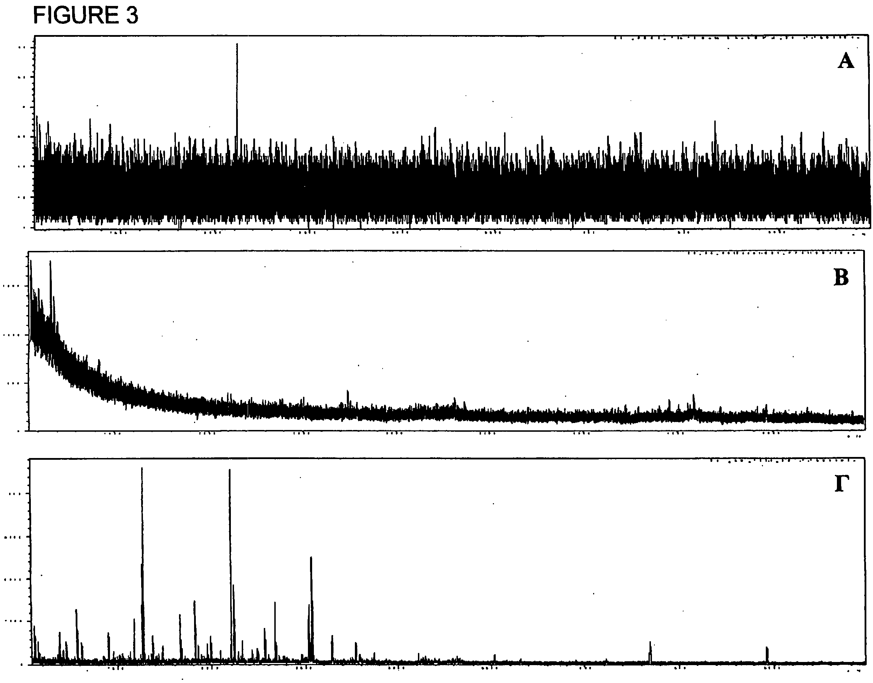

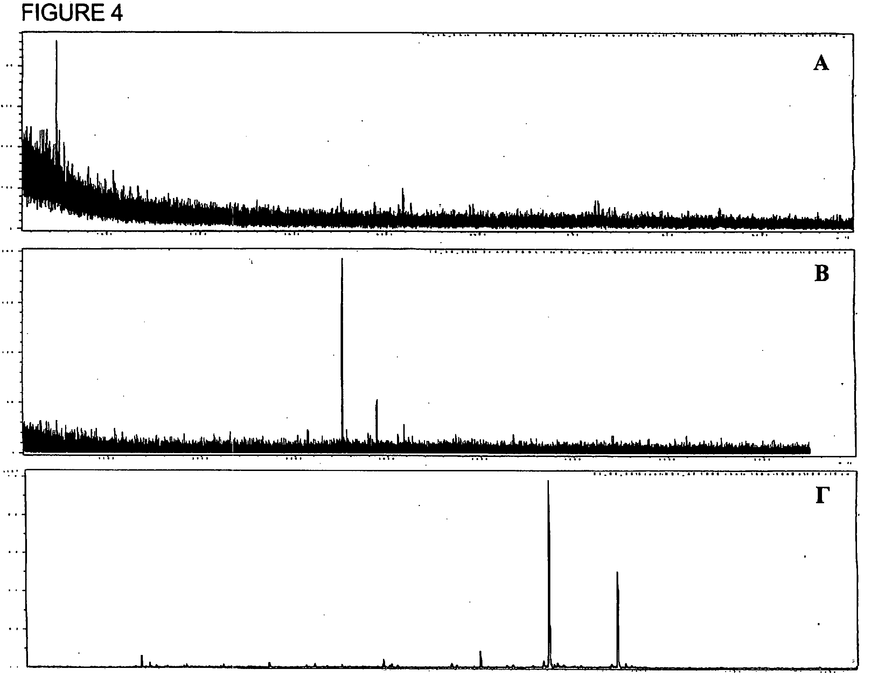

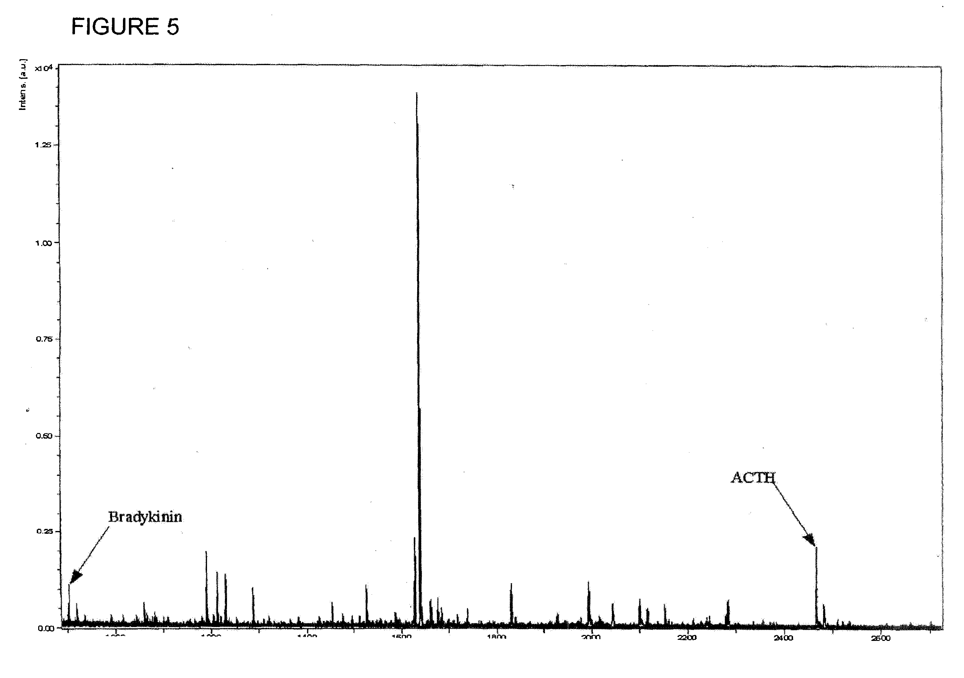

For the evaluation of the effectiveness of the method that is described in the current invention regarding the analysis of NOP/SP, the following experiments were performed.
**a)** One volume of human plasma (a µl) was mixed with 8 volumes (8xa) of aqueous solution 50% acetonitrile, 0.1% TFA and was treated according to the method presented in chapter D.1.A. The pellet and the supernatant are brought back to the original volume (a) using high purity water and were stored for further analysis. In a SDS-PAGE acrylamide gel the following were loaded and analyzed: one volume 5µl of human plasma without and treatment containing 20µg of total protein, 5µl from the pellet solution and equal double and trible the original volume (5, 10, 15µl) from the supernantant solution. As it is evident in figure 1 the method facilitated the release of the NOP/SP from the carrier proteins in solution. Most of the carrier proteins remained in the pellet. Furthmore it is evident that all the proteins with molecular weight higher that 60kDa were totally precipitated.
**b)** One volume of human plasma (b µl) containing 2mg of total protein was mixed with (8xb µl) of aqueous solution 50% acetonitrile, 0.1%TFA and was processed according to the method described in chapter D.1.A. The pellet that is formed from the centrifugation as well as the concentrated supernatant are brought back to the original volume (b µl) with the use of high purity water and are stored for further analysis with the using 2D-SDS gel electrophoresis. For the analysis, half of the initial volume (b/2µl) of the untreated plasma sample containing 1 mg of total protein, equal amount (b/2µl) from the pellet solution and the whole (bµl) supernatant solution are mixed with 250µl sample buffer containing 20mM Tris, 7M Urea, 2M Theiourea, 4% CHAPS, 10mM 1,4 DTE, 1mM EDTA, 1mM PMSF, portease inhibitors, 0.2mM Na₂VO₃ and 1mM NaF. Initially the protein material of these samples is subjected to isoelectric focusing in strips with a non-linear pH range of 3-10 (first dimension). The focusing begins with 250V for 30 minutes and then it rises in a linear way within 14 hours to 6000V. At that point it remains constant for another 18 hours. After the end of the isoelectric focusing the proteins are separated on the basis of their molecular weight (second dimension). The IPG strip carrying the focused proteins is applied to a 12% acrylamide gel. This gels run for 6 hours at 15Watt/gel. After the end of the electrophoresis the gel is fixed in 50%methanol, 5% phosphoric acid for 2 hours and then it is stained with Colloidal Coomassie for 16hours. From the comparison of the gels in figure 2 it is ascertained that the application of the method presented in this invention results in the precipitation of most of the proteins with molecular weight greater than 10kDa. In the supernantant a small percentage of the above mentioned proteins remain as well as most of the proteins with molecular weight less than 10kDa. These small proteins are not possible to be detected with the gel electrophoresis because of technique limitations.
**c)** One volume of human plasma (γ µl) was mixed with 8 volumes of aquaeous solution 50% acetonitrile, 0.1%TFA (8xγ µl) and was treated according to the method presented in chapter C.1.A. 3µl of untreated plasma, 3µl of concentrated supernatant without desalting and 3µl of desalted supernatant (as described in D.1.A.) were placed on a steel MALDI target. After the addition of the matrix solution (D.1.A.) the samples were analyzed with mass spectrometry MALDI-TOF. From the acquired spectra which are presented in Figure 3, it is concluded that NOP/SP cannot be analyzed using mass spectrometry MALDI-TOF neither on the untreated plasma nor on the non-desalted concentrated supernatant. The full application of the method presented in this invention had as a result the acquisition of a mass spectrum bearing a lot of high intensity peaks making the analysis of the human plasma NOP/SP possible.
**d)** One volume of human amniotic fluid (d µl) was mixed with 8 volumes aqueous solution 50% acetonitrile, 0.1 %TFA (8xd µl) and was treated according to the method presented in D.1.B. 3µl of untreated amniotic fluid, 3µl of concentrated supernatant and 3µl of desalted supernatant (as described in D.1.A.) were spotted on a steel MALDI target. The samples were analyzed using mass spectrometry MALDI-TOF. From the acquired spectra which are presented in Figure 4, it is concluded that NOP/SP cannot be analyzed using mass spectrometry MALDI-TOF neither on the untreated amniotic fluid nor on the non-desalted concentrated supernatant. The full application of the method presented in this invention had as a result the acquisition of a mass spectrum bearing a lot of high intensity peaks making the analysis of the amniotic fluid NOP/SP possible.
**e)** In one volume of human plasma (β µl) containing 2mg of total protein, 10pmole of Bradykinin fragment 1-8 (MW: 904.4681) and 20pmole of ACTH fragment 18-39 (MW: 2465.1982) were added and the sample was vortexed. Following that the sample was mixed with 8 volumes of aquaeous solution 50% acetonitrile, 0.1%TFA, it was treated according to the method presented in D.1.A. and analyzed using mass spectrometry MALDI-TOF. The acquired spectrum is presented in Figure 5. The Bradykinin and ACTH fragments are low molecular weight fragments, 904,4681 and 2465.1982 Da respectively. The reason for the execution of this experiment was to determine whether the method described in this invention is capable of isolation low molecular weight proteinic molelcules. As it is evident in the acquired spectrum both Brakykinin and ACTH fragments are detected at the positions corresponding to their molecular weights after treatment of the sample with the method described in this invention.

### D.3. Conclusion

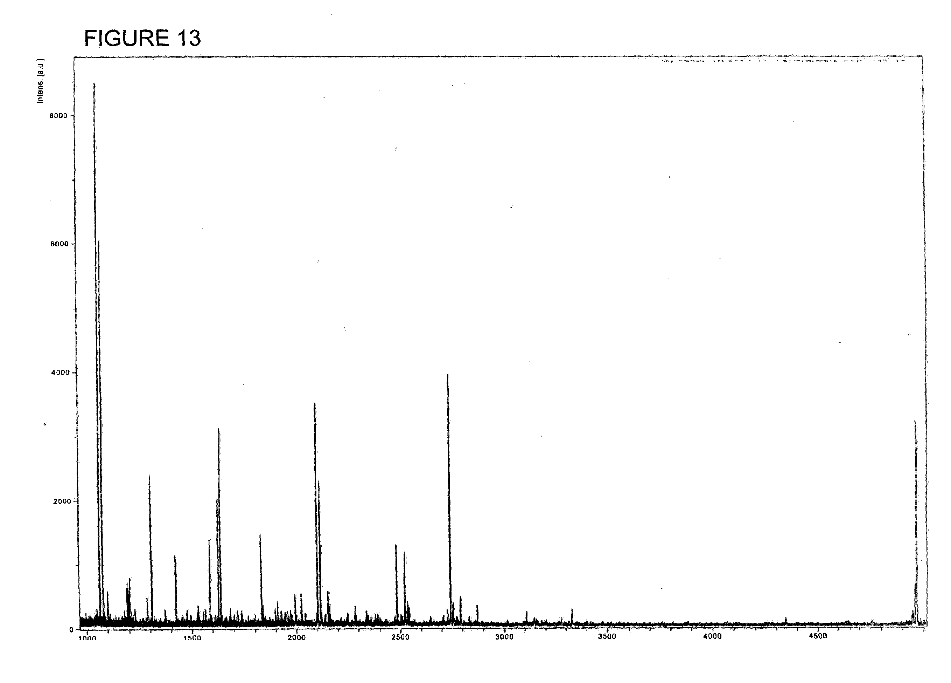

In the current invention an efficient method for the selective analysis of NOP/SP in biological fluids is presented. This method was developed for high protein concentration biological fluids, and low protein concentration biological fluids with minor modifications. The current method was applied for the analysis of NOP/SP in human plasma (high protein concentration biological fluid case) and in aminotic fluid (low protein concentration biological fluid case). The application of this method facilitated the release of the small proteinic molecules from carrier proteins (Figure 1) and their selective isolation from the proteins with molecular weight greater than 10kDa (Figure 2). The NOP/SP were detected for the first time as naturally occuring molecules using MALDI-TOF and ESI-TOF (Q-TOF) mass spectrometry. The aquired spectra are presented in the Figures 13, 14 and 15 in which very peak corresponds to one NOP/SP.

### D.4. Parameters studied for the method development.

### D.4.1. Biological fluid - Acetonitrile,TFA solution mixing ratio.

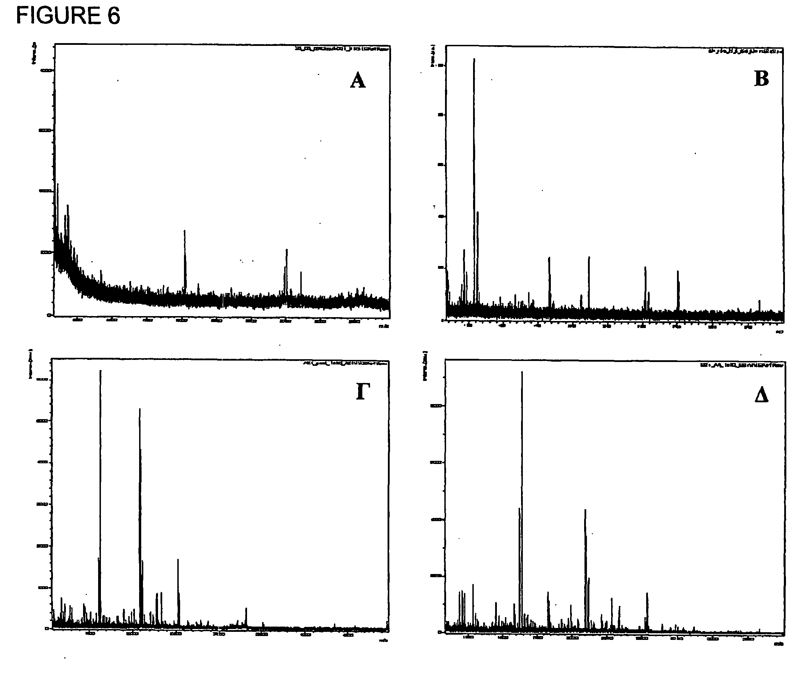

For the determination of the most appropriate mixing ratio of the biological fluid and the 50% acetonitrile, 0.1%TFA solution for the NOP/SP analysis, one volume of human plasma was mixed with 6 to 11 volumes of 50% acetonitrile, 0.1%TFA solution. The NOP/SP analysis method was applied to each sample as described in chapter D.1.A. Mixing one volume of human plasma with 10 and 11 volumes of the 50% acetonitrile, 0.1 %TFA solution did not produce any pellet after the centrifugation step. This indicates that the mixing ratios are inadequate from protein precipitation. Mixing one volume of human plasma with 6 volumes of the 50% acetonitrile, 0.1%TFA solution produced pellet after the centrifugation. Nevertheless, the spectrum produced by MALDI-TOF was very poor in terms of number of peaks and signal intensity. This finding indicates that in the supernatant the amount of NOP/SP was very low, possibly because of precipitation during centrifugation (Figure 6A). Mixing one volume of human plasma with 7, 8, or 9 volumes of the 50% acetonitrile, 0.1%TFA solution resulted in the production of adequate pellet as well as very rich and high quality spectrums in terms of number of peaks and signal intensity as produced my MALDI-TOF analysis. The best results among the above three volumes were produced by mixing one volume of human plasma with 8 or 9 volumes of the 50% acetonitrile, 0.1%TFA solution (Figures 6B, 6C, 6D).

### D.4.2. Acetonitrile concentration in the acetonitrile-TFA solution.

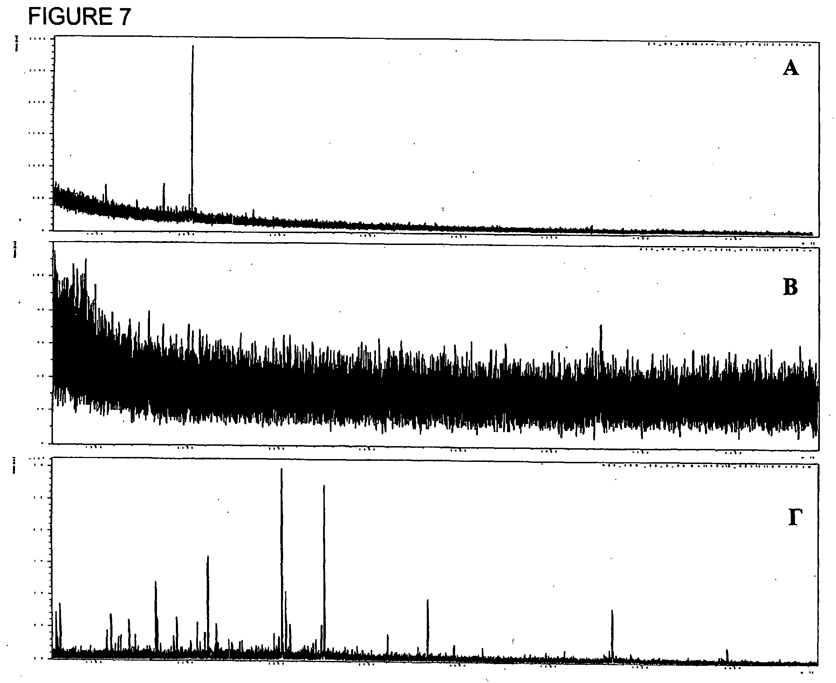

For the determination of the most appropriate acetonitrile quantity in the aqueous solution of acetonitrile ― TFA the following solutions were prepared: a) 40% acetonitrile, b) 45% acetonitrile c) 50% acetonitrile, d) 60% acetonitrile, e) 90% acetonitrile. HPLC purity grade acetonitrile was used for the preparation of all the above solutions. All the solutions contained 0.1%TFA. Nine volumes from each one of these solutions were mixed with one volume of human plasma and the NOP/SP analysis method described in D.1.A. was performed. The mixture of human plasma with the 40 and 45% acetonitrile concentration solutions did not produce any pellet after the centrifugation. The mixture of human plasma with the 60 and 90% acetonitrile concentration solutions produced pellet after the centrifugation, but the spectrums from the MALDI-TOF were very poor, fact that indicates that the concentration of the NOP/SP in these supernatants was very low, possibly because of precipitation during centrifugation (Figures 7A and B). The mixture of human plasma with the 50% acetonitrile concentration solution produced adequate pellet after the centrifugation, while the spectrum produced was rich and high quality in terms of number of peaks and signal intensity (Figure 7C).

### D.4.3. TFA concentration in the acetonitrile-TFA solution

For the determination of the most appropriate TFA concentration in the aqueous solution of acetonitrile-TFA the following solutions were prepared: a)50% acetonitrile - 0.1%TFA b) 50%acetonitrile ― 0.3%TFA. HPLC grade acetonitrile was used for the preparation of all the solutions. Nine volumes of each one of these solutions were mixed with one volume of human plasma and the NOP/SP analysis method described in D.1.A. was performed. The mixture of the solution containing 0.1%TFA produced adequate pellet after the centrifugation and the spectra produced by the MALDI-TOF analysis were rich and of high quality (Figure 7C). The mixture of the solution containing 0.3%TFA did not produce any pellet after centrifugation.

### D.4.4. Time and mixture abode conditions

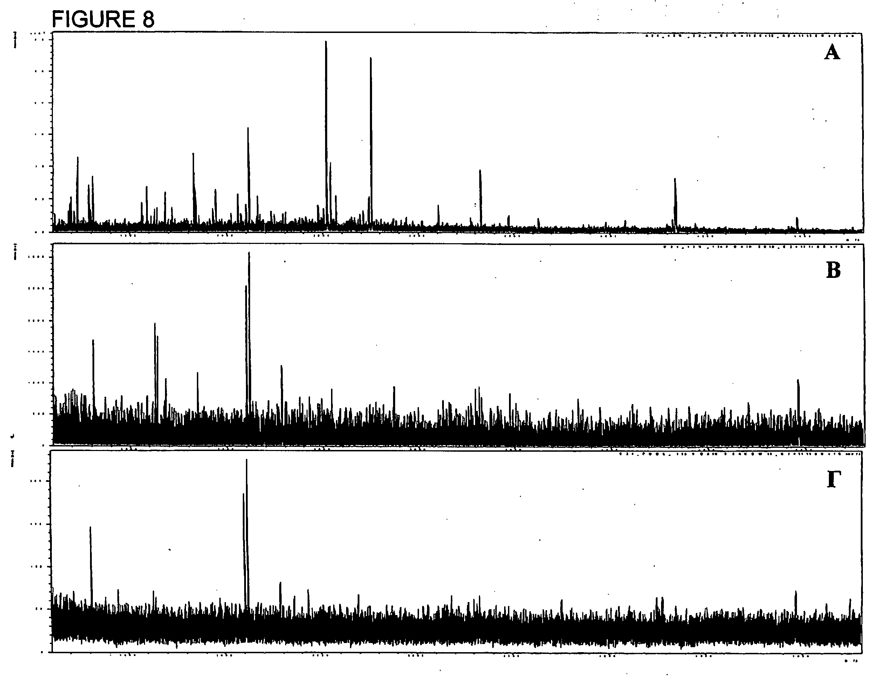

For the determination of the most appropriate conditions (time and temperature) of sample abode prior to centrifugation the following experiments were performed: a) 1 volume of human plasma was mixed with 9 volumes of 50% acetonitrile-0.1% TFA solution, the mixture was vortexed for 5 seconds and was immediately centrifuged at 13.000rpm for 30 minutes at 4°C. The NOP/SP analysis method followed, b) 1 volume of human plasma was mixed with 9 volumes of 50% acetonitrile-0.1% TFA solution, the mixture was vortexed for 5 seconds, remained at room temperature for 30 minutes and then it was centrifuged at 13.000rpm for 30 minutes at 4°C. The NOP/SP analysis method followed, c) 1 volume of human plasma was mixed with 9 volumes of 50% acetonitrile-0.1% TFA solution, the mixture was vortexed for 5 seconds, remained at 4°C for 30 minutes and then it was centrifuged at 13.000rpm for 30 minutes at 4°C. The NOP/SP analysis method followed. NOP/SP from all the above experiments were analyzed by MALDI-TOF. For the acquired spectra (Figure 8), it is evident that the biological fluid ― acetonitrile, TFA solution mixture must be immediately centrifuged after voretxing (Figure 8A).

### D.4.5. Sample centrifugation time

For the determination of the most appropriate sample centrifugation time the following experiments were performed: a) 1 volume of human plasma was mixed with 9 volumes of 50% acetonitrile-0.1% TFA solution, the mixture was vortexed for 5 seconds and was immediately centrifuged at 13.000rpm for 15 minutes at 4°C. The NOP/SP analysis method followed, b) 1 volume of human plasma was mixed with 9 volumes of 50% acetonitrile-0.1% TFA solution, the mixture was vortexed for 5 seconds and was immediately centrifuged at 13.000rpm for 30 minutes at 4°C. The NOP/SP analysis method followed, c) 1 volume of human plasma was mixed with 9 volumes of 50% acetonitrile-0.1% TFA solution, the mixture was vortexed for 5 seconds and was immediately centrifuged at 13.000rpm for 60 minutes at 4°C. The NOP/SP analysis method followed. NOP/SP from all the above experiments were analyzed by MALDI-TOF. From the acquired spectra it is evident that the 15 minute centrifugation time is not sufficient for the separation of the high molecular weight proteins, making the resulting solution inappropriate for desalting. For the isolation of NOP/SP the biological fluid ― acetonitrile,TFA solution mixture must be centrifuged at least for 30 minutes.

### D.4.6. Sample desalting conditions

As already mentioned the mass spectrometric analysis using the MALDI-TOF technology is sensitive to contaminations from organic and inorganic ions. For that reason, sample clean up and desalting prior to MALDI-TOF is a crucial step for a successful analysis. According to the NOP/SP analysis method described in the current invention the protein molecules are eluted from the adsorbent material and directly spotted on the steel target without application of another step of clean up and desalting. For the determination of the most appropriate conditions for sample clean up and desalting the following experiments were performed: a) 1 volume of human plasma was mixed with 9 volumes of 50% acetonitrile-0.1% TFA solution. The sample was treated according to the NOP/SP analysis method described in this invention D.1.A. The sample desalting was performed using C18 material packed in a pipette tip. The C18 material equilibration solution, which was also used for sample washing ― desalting, was 3% acetonitrile, 5% formic acid. The NOP/SP elution from the C18 material was performed using 60% actonitrile, 5% formic acid solution, b) the same procedure as (a) was followed but the NOP/SP elution from the C18 material was performed using 60% acetonitrile, 0.1%TFA, c) the same procedure as (b) was followed but the equilibration of the C18 material was well as the desalting was performed using 3% acetonitrile, 0.1%TFA, d) the same procedure as (c) was followed but the NOP/SP elution from the C18 material was performed using 60% acetonitrile, 0.3%TFA.

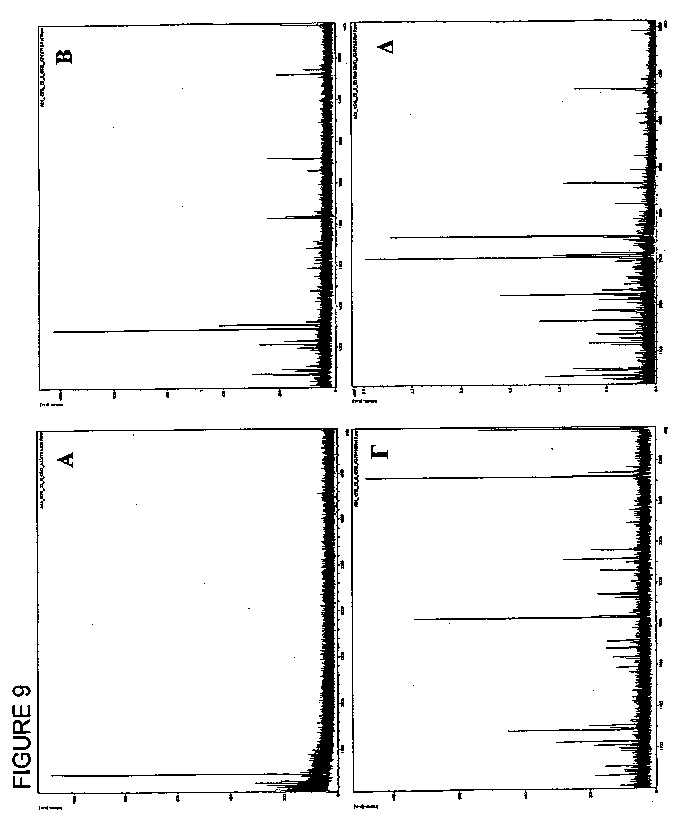

The mass spectrums acquired from each experiment are presented in Figure 9. The best spectrum in terms of low background noise and high number of high intensity peaks was the sample that was desalted with 3%acteonitrile 0.1%TFA and eluted from the C18 material with 60%acetonitrile 0.3%TFA (Figure 9D).

### D.4.7. NOP/SP elution from tip packed with C18 matterial

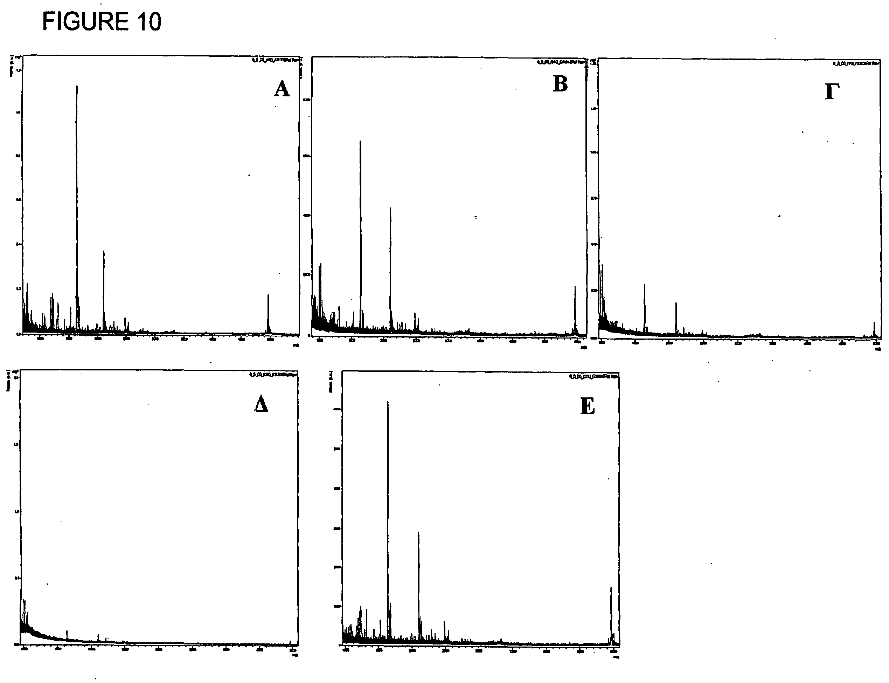

For the determination of the most appropriate NOP/SP elution conditions from the C18 material packed within the pipette tip the following experiments were performed: a) One volume of plasma was mixed with nine volumes of 50%acetonitrile 0.1 %TFA and was treated according to the method presented in the current invention and described in D.1.A. The NOP/SP were eluted four successive times for the tip material using 60%acetonitrile 0.3%TFA. Every elution was spotted on a different steel target position. The four different target spots that corresponded to the four successive elutions were analyzed using the MALDI-TOF and the spectra acquired are presented in Figure 10 (A, B, C and D) b) Aiming for the further enrichment of the NOP/SP experiment (a) was performed but the four successive elutions were spotted on the same steel target position. The spectrum acquired is presented in Figure 10E.

It is established that most of the NOP/SPs are eluted during the first elution. Smaller amounts of NOP/SPs are eluted during the second elution and insignificant amounts are eluted during the third and fourth elution. The attempt to enrich the NOP/SP quantity (Experiment b) did not seem to have a considerable effect.

### D.4.8. Method application to biological fluids with low total protein concentration

Biological fluids with low total protein concentration such as amniotic fluid, urine, cerebrospinal fluid etc. are not only characterized by the low total protein concentration but also by the high concentration of salts, lipids, sugars etc. For the determination of the most appropriate conditions for the isolation of NOP/SP from such biological fluids the following experiments were performed: a) One volume of amniotic fluid was mixed with eight volumes of 50%acetonitrile 0.1%TFA and the NOP/SSP analysis method described in D.1.A. followed, b) One volume of amniotic fluid was mixed with eight volumes of 50%acetonitrile 0.1%TFA and the NOP/SP analysis method described in D.1.B. followed, c) in one volume of amniotic fluid the appropriate amount of protein inhibitors are added and the sample is concentrated to the one tenth of its original volume. In this sample nine volumes of 50%acetonitrile 0.1%TFA are added and the sample is treated according to the method described in D.1.A.

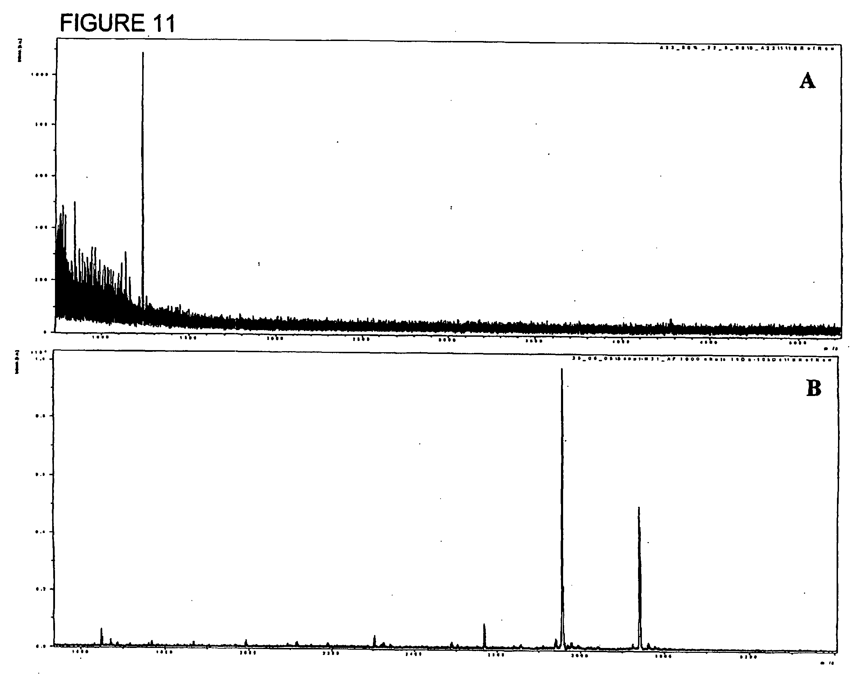

In the first case there was no pellet formed after the centrifugation, fact that indicates that the proteins with molecular weight greater than 10kDa were not precipitated. In the second and third case the samples were analyzed with MALDI-TOF and the spectra acquired are presented in Figure 11. It was observed that the addition of protease inhibitors produces the appearance of a great number of huge intensity peaks in the spectrum obtained by MALDI-TOF which suppressed the NOP/SP peaks (Figure 11A). On the contrary the spectrum obtained from the second experiment produced a rich spectrum in terms of number of peaks corresponding to different NOP/SPs and of peak intensity (Figure 11 B).

### E. Advantages of the current invention

### E.1. NOP/SP isolation

As evident from Figures 1 and 2, the current method application seems to result in the NOP/SP separation from the rest of the biological fluids proteins with molecular weight greater than 10kDa unlike the rest of the methods which analyze NOP/SP as part of the low molecular weight proteome. The separation of the NOP/SPs from the rest of the biological fluids proteins constitutes the greatest advantage of the current method because after the separation, the molecules can be analyzed by all the available mass spectrometry technologies (MALDI-TOF, ESI-TOF etc.). A further advantage of the current method is that it does not include an enzymatic digest as a prerequisite for the NOP/SP identification. Because of that NOP/SP can be analysed as naturally occurring molecules present in the biological fluids and not as part larger protein molecules. The method application presented in the current invention can provide valuable information for the NOP/SP functionality in the biological fluids and contribute towards the investigation of low molecular weight biological markers related to various pathological conditions.

### E.2. Method effectiveness for NOP/SP analysis in comparison to other methods already in use.

In contradiction to the already used methods for the analysis of the biological fluids low molecular weight proteome, the originality of the current invention impress to the fact that NOP/SP are separated from the rest of the protein molecules with molecular weight greater than 10kDa. For the securitization of the advantages of the method presented in the current invention concerning the selective analysis of NOP/SP in biological fluids in relation to the methods already in use, the following experiments were performed: **a)** 1 volume of human plasma was processed with 8 volumes of 50% acetonitrile, 0.1%TFA as described in the method presentation on D.1.A. **b)** the same volume of human plasma was mixed with 2 volumes of pure acetonitrile containing 0.1%TFA. The mixture was treated according to the methodological approach 5 (Chapter C.3.A.5). **c)** the same volume of human plasma was mixed with 5 volume of 25mM NH₄HCO₃, 20% acetonitrile, pH 8,2 and the mixture was placed in an ultracentrifugation filter with molecular weight cut off 10kDa and procedure described in methodological approach 1 was performed(Chapter C.3.A.1.).

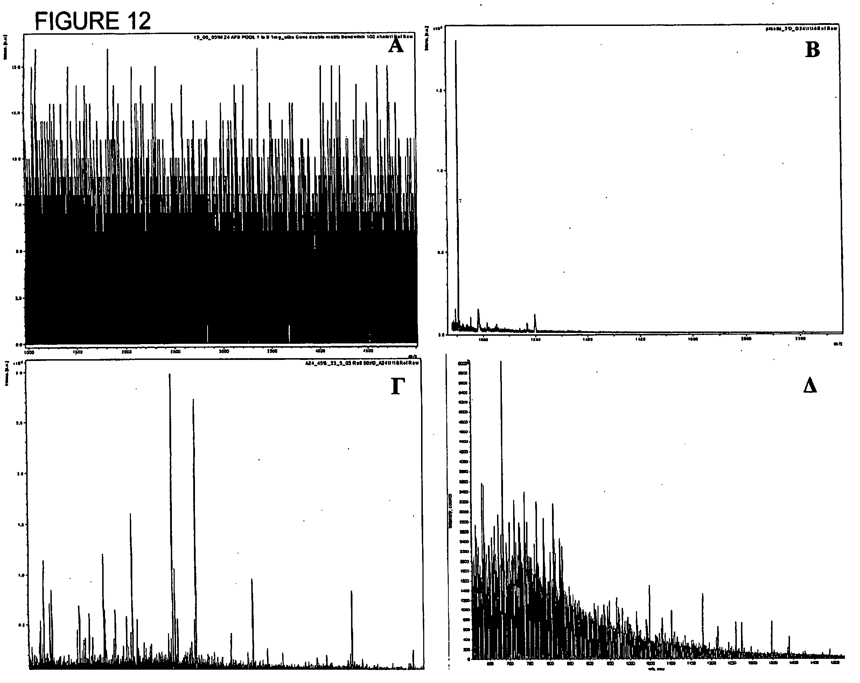

The samples obtained from each experiment were analyzed with MALDI-TOF and ESI-TOF and the spectra acquired are presented in Figure 12. The spectrum acquired from experiment (b) indicates that the quantity of the NOP/SP into solution after the treatment was very low and inadequate for analysis since the intensity of the detected peaks was very low and close to the signal noise (Figure 12A). The spectrum acquired from experiment (c) presented the same low intensity peaks plus the detection of high quantities of plasticizers of various molecular weights which made the analysis of NOP/SP impossible since the high intensity peaks corresponding to the plasticizer molecules were masking the detection of NOP/SPs (Figure 12B). The spectrum acquired from the sample treated with the method presented in the current invention was rich in terms of peak number and peak intensity (Figure 12C). The same results were obtained by the use of the ESI-TOF technology (Figure 12D). It is concluded from spectra evaluation and comparison that the method presented in the current invention is the most efficient on for the analysis of NOP/SP in biological fluids using mass spectrometry. It became evident that for the analysis of the same molecules using the other methods further enrichment and fractionation steps using chromatography methods are required.

### E.3. MALDI-TOF analysis potential

In contradiction to the rest of NOP/SP analysis and identification methods, the method presented in the current invention provides the important potential for the analysis of these molecules with MALDI-TOF mass spectrometry. As mentioned above the particular technology is high throughput and combines high resolution with high intensity levels. The MALDI-TOF technology provides the potential for analysis of multiple samples since the analysis time per sample is less than a minute, the only disadvantage being that it is sensitive to contamination from organic and inorganic ions. This contamination affects the signal significance since a lot of background contaminant peaks are introduced in the spectrum. For the accurate evaluation of a signal the proteins being crystallized on the steel MALDI target must firstly be as contaminant-free as possible and secondly the NOP/SP must be present in such a quantity so as to provide a signal with at least double the noise intensity (signal/noise=2).

From the results acquired from the application of the specific method in a wide range of biological fluids, it is evident that the quality and purity of NOP/SPs present in the sample that is spotted on the MALDI steel target is adequate to provide NOP/SP detection evaluation and identification. From the comparison of the current method with the rest of the methods (Chapter E.2.), it is conclude that the NOP/SP analysis method described in the current invention is the only one suitable for the analysis of these molecules with MALDI-TOF mass spectrometry.

### E.4. Rapid

The total time required for a full analysis of a sample with the method described in the current invention is approximately 1 hour when NOP/SP are analyzed with MALDI-TOF mass spectrometry. The 1 hour time is primarily required for sample preparation (centrifugation, concentration, desalting) and is substantially less than the time required by the methods currently used which include centrifugal ultrafiltration and enzymatic digest of the protein molecules prior to analysis. Furthermore because of the use of MALDI-TOF for NOP/SP analysis, the current method is substantially fast than the methods currently used since there is no sample chromatographic separation prior to mass spectrometry, which is a particularly time consuming step.

### E.5. High throughput

Because of the reliable NOP/SP analysis that can be performed with MALDI-TOF mass spectrometry, the method can be characterized as high throughput. The currently used methods for the NOP/SP analysis have the limitation of analyzing one sample at a time using liquid chromatography prior to mass spectrometry. The method described in the current invention, because of the MALDI-TOF analysis, provides the potential of analysis 384 sample in less than 2 hours.

### E.6. Subject to automation

A great advantage of the MALDI-TOF technology as compared to other mass spectrometry technologies is the potential for automation. This potential is provided by by the use of specialized robotic systems for sample treatment and spotting on MALDI targets. By the use of these systems it is possible for 8 up to 384 samples to be treated and simultaneously spotted on a MALDI steel target. Because of the use of MALDI-TOF technology for the NOP/SP analysis, the method described in the current invention is subject to automation. In particular the tips used for the concentrated sample desalting and clean up can be placed on robotic systems in order for the sample desalting, spotting on the steel target and mixing with the matrix solution to be carried out automatically. Such robotic systems are already commercially available providing the capability of simultaneous handling of 8 to 384 samples and have been already widely utilized in parallel with the MALDI-TOF technology. The sample handling concerning its spotting on the steel target has to be particularly acqurate since the volume being handled is very small and the sample positioning on the target is very tightly defined. The use of robotic systems would therefore facilitate the analysis reducing the preparation time and eliminating errors for a human operator.

### E.7. Large initial biological fluid volume treatment and multiple simultaneous analysis potential

Relevant studies have demonstrated that peptides and low molecular weight proteins originating in tissues and biological fluids can be analyzed utilizing mass spectrometry with minor or even no treatment by simply spotting the sample on the MALDI target and mixing it with matrix solution, or in the case of ESI-TOF by performing a simple buffer exchange in the infusion buffer (60%acetonitrile, 5% formic acid). [Baggerman G, Verleyen P, Clynen E, Huybrechts J, De Loof A, Schoofs L., Peptidomics J. Chromatography B 2004, 803:3-16]

Given the biological fluids complexity which might contain several hundreds or even thousands of different low molecular weight protein species, the analysis of the low molecular weight proteome by mass spectrometry adequate sample fractionation is required prior to mass spectrometric analysis. Furthermore, some of the protein species contained in the biological fluids such as peptidic hormones or microbial toxins are of extremely low abundance, sample concentration is therefore required for their detection. Finally naturally occurring peptides and small proteins present in the biological fluids are very often attached to high molecular weight, high abundance carrier proteins such as albumin. Therefore the application of methods which aim to remove the highly abundant proteins of the biological fluids most probably result in the removal of the attached low molecular weight molecules which constitute potential biological markers.

The effectiveness of a method for the analysis of the biological fluids low molecular weight low abundance proteome must fulfill the following conditions: **a)** Sufficient separation of the small protein molecules from the carrier proteins, **b)** Sufficient separation of the NOP/SPs from the rest of the proteins present in the biological fluids, **c)** Capability to analyze protein molecules present at low concentration.

The method presented in the current invention has the advantage of fulfilling all the above conditions. As evident from the results already presented (Chapter D.4.) NOP/SPs separate from the carrier proteins and from the rest of the proteins present in the biological fluids. Furthermore, the use of the MALDI-TOF technique which is characterized by high resolving power and high sensitivity, makes the highly accurate detection of NOP/SPs present in low concentrations possible.

### a) Large initial biological fluid volume treatment

In the methods for the analysis of the biological fluids low molecular weight proteome currently in use, the initial biological fluid volume used for the analysis is determined and restrained by the loading capacity of the ultracentrifugal filters and of the chromatography columns. The analysis of large volumes of biological fluids with these methds is possible through sample aliquoting, simultaneous aliquote treatment and pooling back together prior to mass spectrometric analysis. The current method being independent from ultracentrifugal filtration and chromatography can be applied to any initial volume of biological fluids which is treated and concentrated uniformly. This fact provides the potential for large volume treatment in order of the extremely low abundant NOP/SP to be detected and accurately identified.

### b) Multiple simultaneous NOP/SP concentrated solution analysis

From the current method application, the resulting NOP/SP concentrated solution can be furtherly treated with different methods. Because of the high sensitivity of the mass spectrometry methods, the concentrated NOP/SP solution can be aliquoted and one aliquot to be analyzed with MALDI-TOF-MS, another with ESI-TOF-MS, another with µLC-MS, another one to be furtherly fractionated with liquid chromatography, another fraction to be analyzed by western blotting etc. The simultaneous concentrated NOP/SP solution analysis potential by different methods provides to the current method the advantage of thorough analysis of the biological fluid under study and the detection and identification of even the lowest abundance NOP/SPs.

### E.8. Quantification Potential

The method described in the current invention, opposing to the methods currently in use, possesses the advantage of NOP/SP quantification in the initial biological fluid, given the fact the concentrated NOP/SP solution can be analyzed with various methods. The quantitation can be achieved either by the use of sophisticated software performing signal analysis and protein profile comparison on the acquired spectra, or by the use of special modification reactions (isotopic labelling, adduct introduction reactions etc.), or by the use of internal standards. As it has been already described in the investigation of method effectiveness section, a defined quantity of one or more peptides of known molecular weight (Bradykinin and ACTH fragments) can be added to a specific volume of a biological fluid sample prior to its treatment with the acetonitrile,TFA solution, so as for the particular peptides to be detected in the resulting spectrum (Chapter D.2.E.). The NOP/SP quantitative determination in the original biological fluid sample can be achieved through one of the quantitative analysis methods for an example through a standard curve of the internal standards correlating concentration to peak intensity and then exalting the NOP/SP concentrations based on than curve.

Internal standards can be added to other parts of the procedure apart from the initial biological fluid sample in order for NOP/SPs to be quantitated such as to the concentrated NOP/SP solution or to the matrix solution. The NOP/SP quantitation in the biological fluid can be achieved through one of the quantitative analysis methods (eg. Standard curve, peak intensity comparison) by exaltation.

### E.9. Advantages summary

As opposed to the currently used methods for the biological fluids low molecular weight proteome analysis, the originality of the current invention lies to the fact that NOP/SPs are separated from the rest of the protein molecules with molecular weight greater than 10kDa and can be selectively analyzed using mass spectrometry as naturally occurring molecules.

The specific method does not include an enzymatic digest, nor any complicated chromatographic separations, is rapid, reproducible and subject to robotic automation. All these facts constitute the method described in the current invention as high throughput. The protein molecules separated with the use of this procedure can be detected, analyzed and identified with any of the available mass spectrometry methods including MALDI-TOF MS/MS without any limitations. Furthermore because of the low number of processing steps, the losses of the low abundant protein molecules present in the biological fluids is minimized.

### F. Figure Description

**Figure 1****.** Representative image of 1 D acrylamide gel in which the following were analysed: 1 volume of 5µl unproccessed human plasma containing 20µg of total protein(A), equal volume from the pellet solution (B) and equal, double and five times the volume (5µl, 10µl and 15µl) from the supernantant solutionn (C,D and E respectively), according to the method described in Chapter D.2.a. M:protein marker
**Figure 2****.** A: Representative image of a polyacrylamide gel containing human plasma according to the method described in Chapter D.2.b. B: Representative image of a polyacrylamide gel containing the pellet obtained from the centrifugation according to the method described in Chapter D.2.b. C: Represeantative image of a polyacrylamide gel containing the supernantant obtained from the centrifugation according to the method described in Chapter D.2.b.
**Figure 3****.** MALDI-TOF mass spectrometric analysis of human plasma according to the method described in Chapter D.2.c. A: Represeantative untreated human plasma spectrum, B: Representative spectrum of concentrated but non-desalted supernantant, C: Representative spectrum of treated human plasma accroding to the method desrcribed in the current invention (Chapter D.1.A.).
**Figure 4****.** MALDI-TOF mass spectrometric analysis of human amniotic fluid according to the method described in Chapter D.2.d. A: Representative spectrum of untreated aminotic fluid, B: Representative spectrum of concentrated but non-desalted supernantant, C: Representative spectrum of treated human plasma accroding to the method desrcribed in the current invention (Chapter D.1.B.).
**Figure 5****.** MALDI-TOF mass spectrometric analysis of human plasma where a Bradykinin fragment (MW:904.4681) and an ACTH fragment (MW:2465.1982) have been added and the sample has been treated according to the method described in Chapter D.2.e. The arrows indicate the Bradykinin and ACTH positions on the final spectrum.
**Figure 6****.** Investigation of the mixing ratio of the biological fluid and the acetonitrile-TFA solution as described in Chpater D.4.1. A: Representative spectrum obtained after mixing 1 volume of human plasma with 6 volumes of acetonitrile-TFA solution, B: Representative spectrum obtained after mixing 1 volume of human plasma with 7 volumes of acetonitrile-TFA solution, C: Representative spectrum obtained after mixing 1 volume of human plasma with 8 volumes of acetonitrile-TFA solution, D: Representative spectrum obtained after mixing 1 volume of human plasma with 9 volumes of acetonitrile-TFA solution.
**Figure 7****.** Investigation of the acetonitrile concentration in the actetonitrile-TFA solution as described in Chapter D.4.2. A: Representative spectrum obtained after mixing 1 volume of human plasma with 9 volumes of 60%acetonitrile-TFA solution, B: Representative spectrum obtained after mixing 1 volume of human plasma with 9 volumes of 90%acetonitrile-TFA solution, and C: Representative spectrum obtained after mixing 1 volume of human plasma with 9 volumes of 50%acetonitrile-TFA solution.
**Figure 8****.** Investigation of time and abode condition of the mixture as described in Chapter D.4.4. A: Representative spectrum accuired from the application of the method described in the current invention (D.1.A.), B: Representative spectrum acuired from a human plasma-acetonitrile TFA solution mixture which remained at room temperature for 30 minutes and then was treated with the current method, C: Representative spectrum acuired from a human plasma-acetonitrile TFA solution mixture which remained at 4°C for 30 minutes and then was treated with the current method.
**Figure 9****.** Investigation of sample desalting conditions as described in Chapter D.4.4. A: Representative spectrum acuired from sample after being desalted with the use of 3% acetonitrile, 5% formic acid solution and eluted from the packed tip with the use of 60%acetonitrile, 3%formic acid, B: Representative spectrum acuired from sample after being desalted with the use of 3% acetonitrile, 5% formic acid solution and eluted from the packed tip with the use of 60%acetonitrile, 0.1%TFA, C: Representative spectrum acuired from sample after being desalted with the use of 3% acetonitrile, 0.1%TFA solution and eluted from the packed tip with the use of 60%acetonitrile, 0.1%TFA, D: Representative spectrum acuired from sample after being desalted with the use of 3% acetonitrile, 0.1%TFA solution and eluted from the packed tip with the use of 60%acetonitrile, 0.3%TFA.
**Figure 10****.** Investigation of NOP/SP elution from the matterial packed within the tip used for desalting as described in chapter D.4.7. The plasma sample is proccessed accoring to the method presented in the current invention (Chapter D.1.A.). NOP/SP are eluted from the tip four sequencial times. Each time the elution product is spoted on a different place on the steel MALDI target. A: Representative spectrum acuired from the first elution, B: Representative spectrum acuired from the second elution, C: Representative spectrum acuired from the third elution, D: Representative spectrum acuired from the fourth elution, E: Representative spectrum acuired from a spot elution where all the elutions had been overlayed.
**Figure 11****.** Investigation of the current invention's method application in the case of biological fluids with low total protein concentrationas described in Chapter D..4.8. A: Representative spectrum aquired after the addition of protease inhibitors in a volume of amniotic fluid, sample concentration to one tenth of the initial volume and sample proccessing accorind to the method presented in the current invention (Chapter D.1.A.). B: Representative spectrum aquired from an amniotic fluid sample treated with the method described in Chapter D.1.B.
**Figure 12****.** Method efficiency for NOP/SP analysis in comparison to other methods currently in use. A: Representative MALDI-TOF mass spectrum acquired after proccessing one volume of human plasma with the methodological approach 5 (Chapter C.3.A.5.), B: Representative MALDI-TOF mass spectrum acquired after proccessing one volume of human plasma with the methodological approach 1 (Chapter C.3.A.1.), C: Representative MALDI-TOF mass spectrum acquired after proccessing one volume of human plasma with the method presented in the current invention, D: Representative ESI-TOF mass spectrum acquired after proccessing one volume of human plasma with the method presented in the current invention.
**Figure 13****.** Representative NOP/SP MALDI-TOF mass spectrum acquired after proccessing one volume of human plasma with the method presented in detail in Chapter G.1.

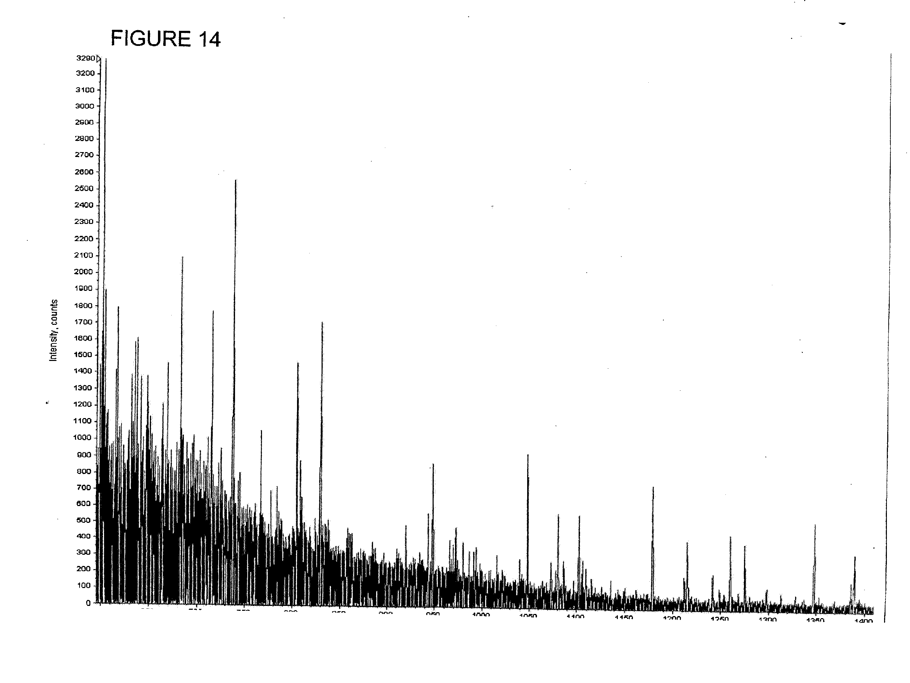

**Figure 14****.** Representative NOP/SP ESI-TOF mass spectrum acquired after proccessing one volume of human plasma with the method presented in detail in Chapter G.2.

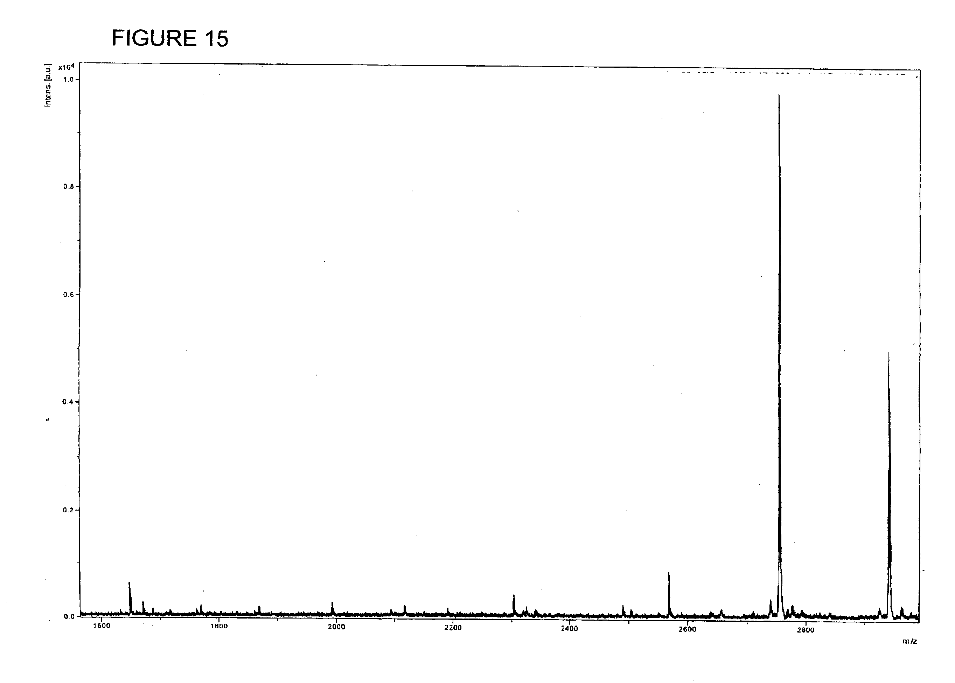

**Figure 15****.** Representative NOP/SP MALDI-TOF mass spectrum acquired after proccessing one volume of human amniotic fluid with the method presented in detail in Chapter G.3.

### G. IN DETAIL PRESENTATION OF A WAY TO APPLY THIS INVENTION

### G.1. Human plasma NOP/SP analysis using MALDI-TOF mass spectrometry.

For NOP/SP analysis human plasma of total protein concentration of 76.01mg/ml was used. In 26.31µl of plasma containing aprroximately 2mg of total protein, 8 volumes (210.48µl) of 50%acetotnitrile, 0.1%TFA solution (purity grade at least HPLC)(solution components ratio:50 parts acetonitrile, 49.9 parts water, 0.1 parts TFA) are added. The sample is vortexed for 5 seconds and immediately centrifuged at 13,000rpm (15,000g) for 30 minutes at 4°C. After the end of the centrifugation the supernantant is transferred to a new tube and is concentrated by centrifugation under vaccum to a volume of 20 µl. Prior to mass spectrometry analysis the sample is desalted using a pipette tip packed with C18 matterial. Prior to sample desalting the C18 matterial is washed 3 times by pipetting up and down 10µl of 100%acetonitrile in water and 3 times by pipetting up and down 10µl of 50%acetonitrile in water. Following that the matterial is equilibrating by 5 passes of 3%acetonitrile, 0.1%TFA solution (volume 10µl). The concentrated sample is pipetted up and down 30 times in order for the NOP/SP to be bound to the matterial. The bound NOP/SPs are then washed by 15 passes of 3%acetonitrile, 0.1%TFA solution (volume 10µl) and then eluted directly on the MALDI target using 3µl of 60%acetonitrile, 0.3%TFA in water. The eluted NOP/SPs are immediately mixed with 1µl of matrix solution [0.3%(w/v) a-Cyano-4-hydroxycinamic acid, 50%(v/v) acetonitrile, 0.1%(v/v)TFA] on target and are allowed to dry. After the mixture has dried 1µl of matrix solution is added on top of the mixture and is again allowed to dry. After this procedure the sample is placed in the mass spectrometer (MALDI ULTRAFLEX, Bruker Daltonics) for the analysis. The analysis is carried out for a molecular weight range of 1000-5200Da having the mass spectrometer set in reflector mode, the laser power at 80% and the detector gain set at 4. The final spectrum is the sum of 5 spectra each comprising 200 laser shots (total of 1000 laser shots). The obtained spectrum is presented in Figure 13. The spectrum analysis indicated that the peaks detected corespond to the analysed human plasma NOP/SPs.

### G.2. Human Plamsa NOP/SP analysis using ESI-TOF mass spectrometry

For NOP/SP analysis human plasma of total protein concentration of 76.01mg/ml was used. In 26.31µl of plasma containing aprroximately 2mg of total protein, 8 volumes (210.48µl) of 50%acetotnitrile, 0.1%TFA solution (purity grade at least HPLC)(solution components ratio:50 parts acetonitrile, 49.9 parts water, 0.1 parts TFA) are added. The sample is vortexed for 5 seconds and immediately centrifuged at 13,000rpm (15,000g) for 30 minutes at 4°C. After the end of the centrifugation the supernantant is transferred to a new tube and is concentrated by centrifugation under vaccum to a volume of 20 µl. Prior to mass spectrometry analysis the sample is desalted using a pipette tip packed with C18 matterial. Prior to sample desalting the C18 matterial is washed 3 times by pipetting up and down 10µl of 100%acetonitrile in water and 3 times by pipetting up and down 10µl of 50%acetonitrile in water. Following that the matterial is equilibrating by 5 passes of 3%acetonitrile, 0.1%TFA solution (volume 10µl). The concentrated sample is pipetted up and down 30 times in order for the NOP/SP to be bound to the matterial. The bound NOP/SPs are then washed by 15 passes of 3%acetonitrile, 0.1%TFA solution (volume 10µl) and then eluted in an eppendorf tube using 10µl of 60%acetonitrile, 0.3%TFA in water. 3µl of the eluant are loaded in a special glass tube bearing external metal coating and ending at a capilary-needle. Through this tube the sample is injected-infused in the ESI-TOF mass spectrometer (Qstar, Applied Biosystems) by application of high voltage and backpressure. The sample analysis conditions are the following: infusion rate 5nL/min, ionspray voltage: 1.0kV, curtain gas: 20L/min, orifice plate potential: 50-75V, focusing potential: 220V, pulser frequency: 6.991kHz, pulse duration: 14µsec, ion transfer stage pressure: 2 x 10⁻⁵ torr, TOF analyser presure: 1.7 x 10⁻⁷ torr, collision cell nitrogen pressure: 4 x 10⁻³ torr, multi-channel plate detector: 2300V, voltage applied to the collision cell: 4V, mass range 400-2000amu. The acquired spectrum is presented in Figure 14.

### G.3. Amniotic Fluid NOP/SP analysis using MALDI-TOF mass spectrometry.

For NOP/SP analysis amniotic fluid of total protein concentration of 8.65mg/ml was used. In 231µl of amniotic fluid containing aprroximately 2mg of total protein, 8 volumes (1848µl) of 50%acetotnitrile, 0.1%TFA solution (purity grade at least HPLC)(solution components ratio:50 parts acetonitrile, 49.9 parts water, 0.1 parts TFA) are added. The sample is vortexed for 5 seconds and concentrated by centrifugation under vaccum to until the point the solution becomes turbid (~ volume of 50 µl). The concentrated solution is centrifuged at 13,000rpm (15,000g) for 30 minutes at 4°C and the supernantant is transferred to a new tube. Prior to mass spectrometry analysis the sample is desalted using a pipette tip packed with C18 matterial. Prior to sample desalting the C18 matterial is washed 3 times by pipetting up and down 10µl of 100%acetonitrile in water and 3 times by pipetting up and down 10µl of 50%acetonitrile in water. Following that the matterial is equilibrating by 5 passes of 3%acetonitrile, 0.1%TFA solution (volume 10µl). The concentrated sample is pipetted up and down 30 times in order for the NOP/SP to be bound to the matterial. The bound NOP/SPs are then washed by 15 passes of 3%acetonitrile, 0.1%TFA solution (volume 10µl) and then eluted directly on the MALDI target using 3µl of 60%acetonitrile, 0.3%TFA in water. The eluted NOP/SPs are immediately mixed with 1µl of matrix solution [0.3%(w/v) a-Cyano-4-hydroxycinamic acid, 50%(v/v) acetonitrile, 0.1 %(v/v)TFA] on target and are allowed to dry. After the mixture has dried 1µl of matrix solution is added on top of the mixture and is again allowed to dry. After this procedure the sample is placed in the mass spectrometer (MALDI ULTRAFLEX, Bruker Daltonics) for the analysis. The analysis is carried out for a molecular weight range of 1000-5200Da having the mass spectrometer set in reflector mode, the laser power at 80% and the detector gain set at 4. The final spectrum is the sum of 5 spectra each comprising 200 laser shots (total of 1000 laser shots). The obtained spectrum is presented in Figure 15. The spectrum analysis indicated that the peaks detected corespond to the analysed amniotic fluid NOP/SPs.

## Claims

1. One method for the analysis of one or more or all of the naturally occurring peptides with molecular weight ≤10kDa and/or small proteins with molecular weight ≤10kDa, present in a plasma or serum sample, comprising of: a) one volume of the biological fluid is mixed with 7 to 9 volumes of aqueous solution containing 50% acetonitrile and 0.1%TFA, b) it is centrifuged, c) the supernatant is separated and concentrated, d) desalted and cleaned, e) analyzed with mass spectrometry.

2. One method for the analysis of one or more or all of the naturally occurring peptides with molecular weight ≤10kDa and/or small proteins with molecular weight ≤10kDa, present in an urine, amniotic fluid, cerebrospinal fluid, saliva or tears sample, comprising of: a) one volume of the biological fluid is mixed with 7 to 9 volumes of aqueous solution containing 50% acetonitrile and 0.1%TFA, b) it is concentrated, c) centrifuged and the supernatant is separated, d) desalted and cleaned, e) analyzed with mass spectrometry.

3. One method, where according to Claims 1 and 2, the solution obtained after the end of stage (c), is desalted and cleaned with the use of an appropriate retention material.

4. One method where according to Claim 3, the retention material is packed in a pipette tip or a chromatography column.

5. One method where according to claims 1 to 4, the solution obtained after the end of stages (c) is separated in fractions.

6. One method where according to Claim 5, the fractionation is carried out using chromatography in particular HPLC and/or LC.

7. One method where according to Claims 1 and 2, the analysis includes the separation and/or the identification and/or the qualitative specification and/or the quantitative specification of one or more or all of the naturally occurring peptides with molecular weight ≤10kDa and/or small proteins with molecular weight ≤10kDa.

8. One method where according to Claim 7, the quantitative specification of one or more or all of the naturally occurring peptides with molecular weight ≤10kDa and/or small proteins with molecular weight ≤10kDa is carried out using one or more internal controls.

9. One method where according to Claims 7 and 8, the analysis of one or more or all of the naturally occurring peptides with molecular weight ≤10kDa and/or small proteins with molecular weight ≤10kDa is carried out using specialized algorithms.

10. One method where according to Claims 1 and 2, the mass spectrometry technology is MALDI-TOF, SEDLI-TOF, ESI-TOF, ESI-IT, ESI-FT, ESI-QqTOF and ESI-QqQ.

11. One method where according to Claims 1 and 2, the whole procedure or part of it is carried out by robotic systems.

12. One method, according to Claims 1 to 11, for the detection and analysis of biological markers indicative of pathological conditions.

13. One method, according to Claims 1 to 12, for the diagnosis of pathological conditions through comparison, evaluation and/or analysis of protein patterns and/or profiles.

14. The use of the method, according to Claims 1 to 13, for the detection and analysis of biological markers.

15. The use of the method, according to Claims 1 to 14, for the diagnosis of pathological conditions.

16. The use of the aqueous solution containing 50% acetonitrile and 0.1% TFA in the processing of biological fluids, by mixing 7 to 9 volumes of that solution with one volume of the biological fluid, aiming to the separation and/or the identification and/or the qualitative specification and/or the quantitative specification of one or more or all of the naturally occurring peptides with molecular weight ≤10kDa and/or small proteins with molecular weight ≤10kDa.

17. The use of the aqueous solution containing 50% acetonitrile and 0.1% TFA according to Claim 16, where the biological fluid is plasma, serum, amniotic fluid, cerebrospinal fluid, urine, saliva, tears.

## Patentansprüche

1. Eine methode für die analyse von einem oder mehreren oder allen natürlich vorkommenden peptiden mit einem molekulargewicht von ≤ 10 kDa und/oder kleinen proteinen mit einem molekulargewicht von ≤ 10 kDa, anwesend in einer plasma- oder serum-probe, bestehend aus: a) ein volumen der biologischen flüssigkeit wird mit 7 zu 9 volumen einer wässrigen lösung, enthaltend 50% acetonitril und 0.1%TFA, gemischt, b) wird zentrifugiert, c) der Überstand wird getrennt und konzentriert, d) entsalzt und gereinigt, e) mit massenspektrometrie analysiert.

2. Eine methode für die analyse von einem oder mehreren oder allen natürlich vorkommenden peptiden mit einem molekulargewicht von ≤ 10 kDa und/oder kleinen proteinen mit einem molekulargewicht von ≤ 10 kDa, anwesend in einer urin-, fruchtwasser-, liquor-, speichel-, oder tränen-probe, bestehend aus: a) ein volumen der biologischen flüssigkeit wird mit 7 zu 9 Volumen einer wässrigen lösung, enthaltend 50% acetonitril und 0.1%TFA, gemischt, b) wird konzentriert, c) wird zentrifugiert und der Überstand wird getrennt, d) entsalzt und gereinigt, e) mit massenspektrometrie analysiert.

3. Eine methode, in der nach den Ansprüchen 1 und 2 die lösung, die nach abschluss der stufe (c) erhalten wird, entsalzt und gereinigt wird mit Hilfe eines geeigneten Retentionsmaterials.

4. Eine methode, in der nach Anspruch 3, das retentionsmaterial in einen pipettentip gepackt wird oder eine Chromatographiesäule.

5. Eine methode, in der nach den Ansprüchen 1 bis 4, die lösung, die nach abschluss der Stufe (c) erhalten wird, in fraktionen getrennt wird.

6. Eine methode, in der nach Anspruch 5, die fraktionierung mit chromatographie HPLC und/oder LC ausgeführt wird.

7. Eine methode, in der nach den Ansprüchen 1 und 2, die analyse die trennung und/oder die identifizierung und/oder die qualitative spezifikation und/oder die quantitative spezifikation von einem oder mehreren oder allen natürlich vorkommenden peptiden mit einem molekulargewicht von ≤ 10 kDa und/oder kleinen proteinen mit einem molekulargewicht von ≤ 10 kDa beinhaltet.

8. Eine methode, in der nach Anspruch 7, die quantitative spezifikation von einem oder mehreren oder allen natürlich vorkommenden peptiden mit einem molekulargewicht von ≤ 10 kDa und/oder kleinen proteinen mit einem molekulargewicht von ≤ 10 kDa mit der anwendung von einem oder mehreren internen standards durchgeführt wird.

9. Eine Methode, in der nach den Ansprüchen 7 und 8, die analyse von einem oder mehreren oder allen natürlich vorkommenden peptiden mit einem molekulargewicht von ≤ 10 kDa und/oder kleinen proteinen mit einem molekulargewicht von ≤ 10 kDa mit der anwendung von spezifischen algorithmen durchgeführt wird.

10. Eine methode, in der nach den Ansprüchen 1 und 2, die massenspektrometrie-technologie MALDI-TOF, SELDI-TOF, ESI-TOF, ESI-IT, ESI-FT, ESI-QqTOF, ESI-QqQ ist.

11. Eine methode, in der nach den Ansprüchen 1 und 2, die ganze prozedur oder teil davon mit robotischen systemen durchgeführt wird.

12. Eine methode, nach den Ansprüchen 1 bis 11, für den nachweis und die analyse von biologischen markern, die auf pathologische konditionen hinweisen.

13. Eine methode, nach den Ansprüchen 1 bis 12, für die diagnose von pathologischen konditionen durch vergleich, auswertung und/oder analyse von proteinpattern und/oder profilen.

14. Die anwendung der methode, nach den Ansprüchen 1 bis 13, für den nachweis und die analyse von biologischen markern.

15. Die anwendung der methode, nach den Ansprüchen 1 bis 14, für die diagnose von pathologischen konditionen.

16. Die anwendung der wässrigen lösung, enthaltend 50% Acetonitril und 0.1% TFA, in der aufbereitung von biologischen flüssigkeiten, durch mischen von 7 zu 9 volumen von der lösung mit einem volumen von der biologischen flüssigkeit, zielend auf die trennung und/oder die identifizierung und/oder die qualitative spezifikation und/oder die quantitative spezifikation von einem oder mehreren oder allen natürlich vorkommenden peptiden mit einem molekulargewicht von ≤ 10 kDa und/oder kleinen proteinen mit einem molekulargewicht von ≤ 10 kDa.

17. Die anwendung der wässrigen lösung, enthaltend 50% Acetonitril und 0.1% TFA, nach Anspruch 16, wobei die biologische flüssigkeit plasma, serum, fruchtwasser, liquor, urin, speichel, träne ist.

## Revendications

1. Une méthode pour l'analyse d'une ou plus ou de toutes les peptides d'un poids moléculaire ≤ 10kDa qui se présentent naturellement et/ou de petites protéines d'un poids moléculaire ≤ 10kDa présentes dans un échantillon de plasma ou de sérum composées de : a) un volume de liquide biologique est mélangé à 7/9 volumes de solution aqueuse contenant 50% d'acétonitrile et de 0,1% TFA, b) cela est centrifugé, c) la solution ci-dessus est séparée et concentrée, d) dessalée et purifiée, e) analysée avec une spectrométrie de masse.

2. Une méthode pour l'analyse d'une ou plus ou de toutes les peptides d'un poids moléculaire ≤ 10kDa qui se présentent naturellement et/ou de petites protéines d'un poids moléculaire ≤ 10kDa présentes dans l'urine, du liquide amniotique, du liquide cérébrospinal, de la salive ou de larmes composées de : a) un volume de liquide biologique est mélangé à 7/9 volumes de solution aqueuse contenant 50% d'acétonitrile et de 0,1% TFA, b) cela est concentré c) centrifugé et la solution ci-dessus est séparée d) dessalée et purifiée, e) analysée avec une spectrométrie de masse.

3. Une méthode où selon les prétentions 1 & 2, la solution obtenue à la fin de la phase (c), est dessalée, purifiée au moyen d'un matériel approprié de rétention.

4. Une méthode où selon la prétention 3, le matériel de rétention est conditionné dans une pipette tip ou une colonne chromatographie.

5. Une méthode où selon les prétentions 1 a 4, la solution obtenue à l'issu de la phase (c) est séparée en fractions.

6. Une méthode où selon la prétention 5, le fractionnement s'effectue en utilisant la chromatographie particulièrement HPLC et /ou LC.

7. Une méthode où sur la base des prétentions 1 et 2, l'analyse comprend la séparation et/ou l'identification et/ou la spécification qualitative et/ou quantitative d'une ou plus ou de toutes les peptides d'un poids moléculaire ≤ 10kDa qui se présentent naturellement et/ou de petites protéines d'un poids moléculaire ≤ 10kDa.

8. Une méthode où selon la prétention 7, la spécification quantitative d'une ou plus ou de toutes les peptides d'un poids moléculaire ≤ 10kDa qui se présentent naturellement et/ou de petites protéines d'un poids moléculaire ≤ 10kDa s'effectue en utilisant un ou plusieurs contrôles internes.

9. Une méthode où sur la base des prétentions 7 et 8, l'analyse d'une ou plus ou de toutes les peptides d'un poids moléculaire ≤ 10kDa qui se présentent naturellement et/ou de petites protéines d'un poids moléculaire ≤ 10kDa s'effectue en utilisant des algorithmes spéciaux.

10. Une méthode où selon les prétentions 1 et 2, la technologie spectrométrique de masse est MALDI-TOF, SEDLI-TOF, ESI-TOF, ESI-IT, ESI-FT, ESI-QqTOF, ESI-QqQ.

11. Une méthode où selon les prétentions 1 et 2 l'ensemble de la procédure, ou une partie de celle-ci, s'effectue au moyen de systèmes robotisés.

12. Une méthode sur la base des prétentions 1 à 11, pour la détection et l'analyse de marqueurs biologiques indicatrice de conditions pathologiques.

13. Une méthode sur la base des prétentions 1 à 12 pour le diagnostic de conditions pathologiques à travers la comparaison, l'évaluation et/ou l'analyse de structures (schémas) protéiniques et/ou de profils.

14. L'utilisation de la méthode sur la base des prétentions 1 à 13 pour la détection et l'analyse de marqueurs biologiques.

15. L'utilisation de la méthode sur la base des prétentions 1 à 14 pour le diagnostic de conditions pathologiques.

16. L'utilisation d'une solution aqueuse contenant 50% d'acétonitrile et 0,1% de TFA dans l'élaboration de liquides biologiques en mélangeant 7 à 9 volumes de cette solution avec un volume de liquide biologique dans le but de séparer et/ou d'identifier et/ou de spécifier qualitativement ou quantitativement une ou plus ou de toutes les peptides d'un poids moléculaire ≤ 10kDa qui se présentent naturellement et/ou de petites protéines d'un poids moléculaire ≤ 10kDa.

17. L'utilisation d'une solution aqueuse contenant 50% d'acétonitrile et 0,1% de TFA sur la base de la prétention 16, ou le liquide biologique est du plasma, du sérum, du liquide cérébrospinal, de l'urine, de la salive, des larmes.
